# EUROPÄISCHE PATENTSCHRIFT

(11) **EP 4 615 450 B1**
(45) Veröffentlichungstag und Bekanntmachung des Hinweises auf die Patenterteilung: **27.05.2026**
(21) Anmeldenummer: 24703947.2
(22) Anmeldetag: 05.02.2024
(51) Int. Cl.: A61K 31/444, A61K 45/06, A61P 31/14

(54) **LUTSCHTABLETTENFORMULIERUNG ENTHALTEND OCTENIDIN FÜR DIE BEHANDLUNG VON CORONAVIRUS-INFEKTIONEN**
LOZENGES FORMULATION COMPRISING OCTENIDINE FOR THE TREATMENT OF CORONAVIRUS INFECTIONS
FORMULATION DE PASTILLES COMPRENANT DE L'OCTÉNIDINE POUR LE TRAITEMENT D'INFECTIONS PAR CORONAVIRUS

(30) Priorität: 02.03.2023 DE 102023105149; 02.06.2023 DE 102023114609
(43) Veröffentlichungstag der Anmeldung: 17.09.2025
(73) Patentinhaber: Maria Clementine Martin Klosterfrau Vertriebsgesellschaft mbH, 50670 Köln (DE)
(72) Erfinder: PLOCH, Michael, 50670 Köln (DE)
(74) Vertreter: Strehlke, Ingo Kurt
(86) Internationale Anmeldenummer: PCT/EP2024/052753
(87) Internationale Veröffentlichungsnummer: WO 2024/179785

(56) Entgegenhaltungen:
- WO-A1-2006/039961
- WO-A1-2021/197646
- CN-A- 114 515 286
- K�HNLEIN J ET AL: "Zur antimikrobiellen und viruziden Wirksamkeit von Octenidin-Lutschtabletten", KRANKENHAUSHYGIENE + INFEKTIONSVERH}TUNG, FISCHER, JENA, DE, vol. 38, no. 4, 18 July 2016 (2016-07-18), pages 165 - 173, XP029707343, ISSN: 0720-3373, DOI: 10.1016/J.KHINF.2016.06.001

## Beschreibung

Die vorliegende Erfindung betrifft das Gebiet der Medizin und insbesondere das technische (d. h. medizinisch-pharmazeutische) Gebiet der Behandlung bzw. Therapie von viralen Erkrankungen bzw. von viralen Infektionen (viralen Infektionskrankheiten bzw. Virusinfektionen) insbesondere von durch Corona-Viren, vorzugsweise SARS-CoV-2, hervorgerufenen (Virus-)Erkrankungen, vorzugsweise COVID-19.

Insbesondere betrifft die vorliegende Erfindung eine Zusammensetzung zur Verwendung bei der prophylaktischen bzw. therapeutischen topischen (lokalen) Behandlung von viralen Erkrankungen (synonym auch als "(Virus-)Infektionen" oder dergleichen bezeichnet), nämlich von durch Corona-Viren, vorzugsweise SARS-CoV-2, hervorgerufenen Viruserkrankungen (synonym auch als "Corona-Infektionen", "Corona(virus)-Infektionen" oder dergleichen bezeichnet), insbesondere von COVID-19, bzw. die Verwendung einer Zusammensetzung (zur Herstellung eines Arzneimittels oder Medikaments) zur prophylaktischen bzw. therapeutischen topischen Behandlung der zuvor beschriebenen viralen Erkrankungen bzw. (Virus-)Infektionen. Im Rahmen der vorliegenden Erfindung kommt dabei als antiviraler Wirkstoff Octenidin und/oder dessen Salze und/oder dessen Ester, vorzugsweise Octenidindihydrochlorid, zum Einsatz.

Coronaviridae ist eine Virusfamilie innerhalb der Ordnung Nidovirales. Die Familie der Coronaviren verdankt ihren Namen den Proteinen an der Oberfläche des Viruspartikels, die dem Virus unter elektronenmikroskopischer Betrachtung ein kronenartiges Aussehen verleihen. Die Viren innerhalb dieser Virusfamilie werden fachumgangssprachlich auch Coronaviren genannt und gehören zu den RNA-Viren mit den größten Genomen. Die ersten Coronaviren wurden bereits Mitte der 1960er Jahre entdeckt und beschrieben. Die im elektronenmikroskopischen Bild grob kugelförmigen Viren fallen durch einen Kranz blütenblattartiger Fortsätze auf, die an eine Sonnenkorona erinnern, was dieser Virusfamilie ihren Namen gegeben hat.

Vertreter der Coronaviridae-Virenfamilie verursachen bei allen vier Klassen der Landwirbeltiere (d. h. Säugetiere, Vögel, Reptilien und Amphibien) sehr unterschiedliche Erkrankungen. Sie sind genetisch hochvariabel und können so auch mehrere Arten von Wirten infizieren und zudem neue Virusvarianten ausbilden.

Beim Menschen sind mehrere Arten bzw. Spezies von Coronaviren als Erreger von leichten respiratorischen Infektionen (insbesondere Erkältungen bzw. grippalen Infekten) bis hin zum sogenannten schweren akuten Atemwegssyndrom (SARS bzw. *Severe Acute Respiratory Syndrome*) von Bedeutung. Unter den menschlichen Coronaviren besonders bekannt geworden sind SARS-CoV-1 (*Severe Acute Respiratory Syndrome Coronavirus-1*), MERS-CoV (*Middle East Respiratory Syndrome Coronavirus*) und SARS-CoV-2 (*Severe Acute Respiratory Syndrome Coronavirus 2* bzw. *schweres akutes respiratorisches Syndrom Coronavirus 2* bzw. COVID-19). Diese Coronaviren sind die Auslöser der SARS-Pandemie 2002/2003, der MERS-Epidemie 2012 bzw. der COVID-19-Pandemie ab 2019.

Für die von SARS-CoV-2 im Speziellen ausgelöste Erkrankung hat die Weltgesundheitsorganisation (WHO) den offiziellen Namen COVID-19 festgelegt. COVID-19 (d.h. die Abkürzung für Englisch *Coronavirus-Disease 2019* oder Deutsch Coronavirus-Krankheit-2019, umgangssprachlich auch als Coronavirus-Erkrankung/-Infektion oder dergleichen bezeichnet) ist somit eine Infektionskrankheit, bei welcher es zu einer Infektion mit dem Coronavirus SARS-CoV-2 kommt. Die Erkrankung wurde erstmals Ende des Jahres 2019 in Wuhan/China beschrieben, entwickelte sich dann im Januar 2020 zunächst in der Volksrepublik China zu einer Epidemie und breitete sich schließlich weltweit zur COVID-19-Pandemie aus.

Die Übertragung bzw. Infektion mit SARS-CoV-2 erfolgt im Allgemeinen durch Tröpfchen (Tröpfchenübertragung bzw. Tröpfcheninfektion) bzw. Aerosole, welche beim Sprechen, Husten und Niesen entstehen. Das Virus kann im Hinblick auf die Infektion über die Atemwege, die Augen, die Nase und den Mund- bzw. Rachenraum in den Körper gelangen. Das Ansteckungsrisiko kann durch das Tragen von Mund-Nasen-Bedeckungen, eine konsequente Handhygiene (regelmäßiges Händewaschen) sowie Husten und Niesen in die Armbeuge verringert, jedoch nicht ausgeschlossen werden. Auch das sogenannte *Social Distancing* kann die Übertragung von SARS-CoV-2 reduzieren.

Die Inkubationszeit von SARS-CoV-2 beträgt durchschnittlich fünf bis sechs Tage, wobei zwischen Ansteckung und dem Auftreten erster Symptome bisweilen aber auch bis zu vierzehn Tage vergehen können und vereinzelt erste Symptome schon innerhalb von 24 Stunden nach Ansteckung mit SARS-CoV-2 auftreten können. Die häufigsten Symptome sind Fieber, trockener Husten und Müdigkeit. Weniger häufig sind dagegen Muskelschmerzen, eine verstopfte Nase, Kopfschmerzen, Bindehautentzündung, Halsschmerzen, Durchfall, Geschmacks- oder Geruchsverlust oder Hautausschlag oder Verfärbung von Fingern oder Zehen zu beobachten.

Auch infizierte Personen ohne Symptome oder mit leichtem Erkrankungsverlauf können das Virus weitergeben. Bei einem leichten Verlauf klingen die Symptome im Allgemeinen innerhalb von zwei Wochen ab. Verläuft COVID-19 schwerer, kann die Rekonvaleszenz drei bis sechs Wochen oder sogar noch länger andauern.

COVID-19 kann sich somit auch mit einem allgemeinen schweren Krankheitsgefühl manifestieren. Im weiteren Verlauf kann sich dann eine schwere Atemnot aufgrund einer Infektion der unteren Atemwege bis zur Lungenentzündung entwickeln. Diese kann mit Brustschmerzen im Sinne einer Pleuritis einhergehen. Etwa 85 % der schwer erkrankten COVID-19-Patienten entwickeln eine Lymphopenie. Schwer erkrankte Patienten entwickeln häufig zudem eine Hyperzytokinämie (sogenannter Zytokinsturm), wobei dieser Zytokinsturm durch eine Überreaktion des Immunsystems entsteht; diese Überreaktion ist durch einen deutlichen Anstieg von entzündungsrelevanten Zytokinen gekennzeichnet, wie insbesondere Interleukin-6, Interleukin-8, Interleukin-1beta und TNF-alpha. Die verstärkte Freisetzung dieser Zytokine führt zu einer Überproduktion von Immunzellen, vor allem im Lungengewebe.

Im Allgemeinen liegt im Zusammenhang mit den insgesamt registrierten Infektionen jedoch häufig ein leichter Krankheitsverlauf mit Fieber oder einer leichten Lungenentzündung vor. Bei einem geringeren Anteil liegt jedoch, wie zuvor angeführt, ein schwerer Verlauf vor, welcher sogar mit einer intensivmedizinischen Betreuung bzw. Beatmung betroffener Personen einhergehen kann. Insbesondere bei älteren und immungeschwächten Personen sowie bei Personen mit Vorerkrankungen kann SARS-CoV-2 zu besonders schweren Erkrankungsverläufen führen. Insgesamt geht eine Infektion mit SARS-CoV-2 somit mit unterschiedlichen Krankheitsverläufen im Hinblick auf COVID-19 einher. So können symptomlose Verläufe sowie symptomatische Verläufe, hierunter auch schwere und sogar tödliche Verläufe, vorliegen bzw. auftreten.

Was klinische Symptome und laborchemische Krankheitszeichen zudem anbelangt, so ist eine Abgrenzung von anderen Viruserkrankungen, wie etwa Influenza, allein anhand der Symptome mitunter schwierig. Die Diagnose von COVID-19 kann insbesondere durch labordiagnostischen Nachweis erfolgen, insbesondere mittels spezifischer Virus- und Antikörpernachweise.

Für den der Erkrankung zugrundeliegenden Pathomechanismus ist insbesondere auch der Replikationszyklus von SARS-CoV-2 von Bedeutung. So dringt das COVID-19 auslösende Virus typischerweise über eine Bindung an das in der Zellmembran einer (menschlichen) Wirtszelle verankerte Enzym ACE2 (Angiotensin-konvertierendes Enzym 2) in die Wirtszelle ein, wobei das Virus über sein Spike-Protein mit ACE2 interagiert. ACE2 ist am Abbau von Angiotensin II beteiligt, welches den Blutdruck erhöht und proinflammatorische Effekte zeigt. Durch die Bindung des Spike-Proteins wird die Funktion von ACE2 gehemmt, was entzündliche Reaktionen fördern kann. ACE2 wird durch die Virusinfektion zudem herunterreguliert. Für den Infektionsprozess ist zudem die Mitwirkung der Serinprotease TMPRSS2 (Transmembrane Serinprotease 2) von Relevanz. Diese körpereigene und membranstämmige Protease wird von SARS-CoV-2 für das Eindringen in die Wirtszelle (und die Freisetzung aus der Wirtszelle) benötigt.

Bei der Infektion wird SARS-CoV-2 über Endosomen in die Wirtszelle aufgenommen. Aus ihnen wird die Viren-RNA freigesetzt, welche für die Bildung der viralen Proteine und für die Synthese von neuem Genmaterial benötigt wird. Neu gebildete Viren gelangen wiederum durch Exozytose aus der Wirtszelle und können weitere Zellen infizieren bzw. in die Umgebung abgegeben werden und weitere Personen infizieren.

COVID-19 und der dieser Erkrankung zugrundeliegende Erreger SARS-CoV-2 sind Gegenstand intensiver Forschungen, und zwar auch vor dem Hintergrund, kausale Therapien und Impfungen zur Verfügung zu stellen.

Was die medikamentöse Vorbeugung von COVID-19 anbelangt, so sind zwischenzeitlich effektive Impfstoffe verfügbar, welche einen hohen Beitrag in Bezug auf die Verhinderung einer Infektion, die Verminderung schwerer Krankheitsverläufe und die Kontrolle der Infektionskrankheit bzw. -ausbreitung leisten. Je nach Typ der Impfung werden Antigene oder Nukleinsäuren von SARS-CoV-2 verabreicht (wobei aus den Nukleinsäuren im Körper die korrespondierenden viralen Proteine gebildet werden), was eine Immunantwort auslöst und insbesondere zur Bildung von Antikörpern führt, welche vor der Infektion schützen bzw. die Infektionsgefahr verringern bzw. im Fall einer Infektion zu weniger schweren Krankheitsverläufen führen.

Seit dem weltweiten Ausbruch von SARS-CoV-2 wurde bislang eine Vielzahl an COVID-19-Impfstoffen entwickelt, hierunter sogenannte mRNA-Impfstoffe, DNA-Impfstoffe sowie Impfstoffe auf Basis inaktivierter Coronaviren und sogenannte Untereinheitenimpfstoffe, welche auf Proteinen des Virus basieren. Für die Impfstoffe ist hauptsächlich das Spike-Protein von SARS-CoV-2 von Bedeutung, welches für die Bindung des Virus an die Wirtzelle und die Aufnahme in die Zellen verantwortlich ist, wie zuvor ausgeführt. Im Allgemeinen führen die Impfstoffe zu einer gewissen Immunität gegenüber SARS-CoV-2, wobei auch das Auftreten schwerer Verläufe der Erkrankungen verringert werden kann. Jedoch ist nicht immer ein vollständiger Immunschutz bzw. nur eine bedingte Immunität gegeben, und zwar auch in Bezug auf neu auftretende Virusvarianten oder dergleichen.

Leichte Erkrankungen können symptomatisch, z. B. mit fiebersenkenden Arzneimitteln, Schmerzmitteln, hustenreizlindernden Arzneimitteln bzw. Antitussiva und Expektorantien bzw. Sekretolytika behandelt werden. Weiterhin können abschwellende Nasensprays eingesetzt werden. Im Hinblick auf eine kausale Therapie können antivirale Wirkstoffe zum Einsatz kommen. Hierzu zählen beispielsweise sogenannte RNA-Polymerase-Inhibitoren bzw. Nukleosid-Analoga, wie Remdesivir. Remdesivir ist ein antiviraler Wirkstoff für die Behandlung von COVID-19. Remdesivir hat ein breites Wirkspektrum und ist unter anderem gegen Coronaviren wirksam, wobei die virale RNA-Synthese und die Virusvermehrung gehemmt werden. Das Arzneimittel wird als intravenöse Infusion verabreicht. Weiterhin stehen antivirale Wirkstoffe aus der Gruppe der 3CL-Protease-Inhibitoren für die Behandlung von COVID-19 zur Verfügung, wobei die Wirkung auf der Hemmung der viralen Protease 3CL beruht. Hierdurch wird die Verarbeitung viraler Proteine und Enzyme sowie die Virusvermehrung gestört bzw. gehemmt. Weiterhin können Wirkstoffe in Betracht gezogen werden, welche die Protease TMPRSS2 hemmen (welche, wie zuvor angeführt, für die Aufnahme von SARS-CoV-2 in die Wirtszellen von Bedeutung ist). Weiterhin sind Arzneimittel in Form von Fusionsinhibitoren, monoklonalen Antikörpern oder dergleichen von Bedeutung. Zudem ist auch auf Immunsuppressiva bzw. Immunmodulatoren, wie Dexamethason, abzustellen. Derartige Wirkstoffe können eine übermäßige und körpereigene Immunreaktion im Zusammenhang mit COVID-19 kontrollieren, welche für Symptome und Komplikationen mitverantwortlich ist.

Insgesamt liegen zur Behandlung von COVID-19 zwischenzeitlich somit auch kausale Therapieansätze vor. Diese gehen oftmals jedoch mit unerwünschten bzw. übermäßigen Nebenwirkungen einher und fokussieren maßgeblich auf einen rein systemischen Ansatz mit therapeutischem Schwerpunkt nach Manifestation bzw. Ausbruch der Krankheit und bedürfen oftmals einer intensiven Kontrolle unter enger fachmedizinischer Aufsicht.

Nach gegenwärtigem Kenntnisstand wird sich das Virus SARS-CoV-2 auch nach Abklingen der pandemischen Situation langfristig als endemisches Virus etablieren, so dass dauerhaft in Teilen der Bevölkerung eine Zirkulation des Virus vorliegt. Dies bedeutet, dass auch langfristig noch Infektionen auftreten können, welche weiterhin auch zu schweren Verläufen der COVID-19-Erkrankung führen können. Zudem können sich neue Virusvarianten von SARS-CoV-2 entwickeln, welche sich zudem zumindest teilweise der Schutzwirkung bestehender Impfungen entziehen können.

Daraus ergibt sich insgesamt, dass neben den zur Verfügung stehenden Impfungen, einer umfassenden Diagnostik zur Früherkennung infizierter Personen und den verfügbaren symptomatischen sowie kausalen Therapien weiterführende Wirkstoffe bzw. Behandlungskonzepte erforderlich sind, welche zur Behandlung von COVID-19 bzw. zur Bekämpfung von SARS-CoV-2 eingesetzt werden können.

Die WO 2021/197646 A1 betrifft eine wässrige Zusammensetzung, welche Octenidin oder ein pharmazeutisch annehmbares Salz davon und Phenoxyethanol bzw. Phenoxypropanol in einem speziellen Gewichtsverhältnis bei gleichzeitig spezieller Phenoxyethanol- bzw. Phenoxypropanol-Konzentration umfasst.

Das Dokument gemäß Köhnlein J et al., "Zur antimikrobiellen und viruziden Wirksamkeit von Octenidin-Lutschtabletten", Krankrenhaushygiene und Infektionsverhütung, Fischer, Jena, DE, Bd. 38, Nr. 4, 18. Juli 2016, Seiten 165 bis 173, betrifft Untersuchungen zur antimikrobiellen und viruziden Wirksamkeit von Octenidin.

Die CN 114 515 286 A betrifft die Verwendung von Octenidin zur Herstellung von Arzneimitteln mit antiviraler Wirkung.

Die WO 2006/039961 A1 betrifft eine pharmazeutische Zusammensetzung in Form einer festen Dosierung zum Lutschen zur topischen Behandlung von entzündlichen Erkrankungen des Mund- und Rachenraums, wobei die Zusammensetzung eine therapeutisch wirksame Menge an Octenidin enthalten soll.

Insbesondere besteht somit weiterhin auch ein großer Bedarf an der Bereitstellung neuer bzw. ergänzender Behandlungskonzepte, welche eine gegen das Virus gerichtete Wirksamkeit aufweisen, nebenwirkungsarm sind und zudem mit einer einfachen Handhabung und hoher Anwendungssicherheit einhergehen. Zudem besteht ein großer Bedarf an der Bereitstellung auch präventiver bzw. prophylaktischer Konzepte, auf deren Basis eine Infektion sowie die Weitergabe des Virus unterbunden bzw. die Weitergabe und Infektionsgefahr verringert werden kann. Zudem liegt auch ein Bedarf an der Bereitstellung entsprechender Wirkstoffe vor, welche co-therapeutisch mit bekannten Wirksubstanzen eingesetzt werden können.

Vor diesem Hintergrund besteht eine Aufgabe der vorliegenden Erfindung darin, einen effizienten (Behandlungs-)Ansatz gegenüber durch Corona-Viren, insbesondere SARS-CoV-2, ausgelösten Viruserkrankungen bzw. gegenüber durch Corona-Viren ausgelösten viralen Infektionen (Virusinfektionen), insbesondere COVID-19, bereitzustellen, wobei auch die zuvor geschilderten Nachteile des Standes der Technik zumindest weitgehend vermieden oder wenigstens abschwächt werden sollen.

Der vorliegenden Erfindung liegt dabei insbesondere die Aufgabe zugrunde, einen geeigneten Wirkstoff und eine diesen Wirkstoff enthaltende Zusammensetzung aufzufinden bzw. bereitzustellen, welcher bzw. welche sich jeweils zur effizienten prophylaktischen bzw. therapeutischen Behandlung von viralen Erkrankungen, welche insbesondere durch Corona-Viren (d. h. Corona(virus)infektionen), vorzugsweise SARS-CoV-2, hervorgerufen werden, eignen, und zwar bevorzugt im Rahmen einer effizienten Prophylaxe bzw. Therapie.

Insbesondere ist eine weitere Aufgabe der vorliegenden Erfindung auch darin zu sehen, einen geeigneten Wirkstoff und eine diesen Wirkstoff enthaltende Zusammensetzung aufzufinden bzw. bereitzustellen, welcher bzw. welche sich zur Verwendung bei der prophylaktischen bzw. therapeutischen Behandlung von viralen Erkrankungen, insbesondere von durch Corona-Viren, vorzugsweise SARS-CoV-2, hervorgerufenen (Virus-)Erkrankungen, bevorzugt von COVID-19, eignet/eignen.

In diesem Zusammenhang liegt eine nochmals weitere Aufgabe der vorliegenden Erfindung auch darin, einen entsprechenden Wirkstoff bzw. eine diesen Wirkstoff enthaltende Zusammensetzung aufzufinden bzw. bereitzustellen, wobei bei hoher Wirksamkeit gegenüber den in Rede stehenden Viren auch eine einfache Verabreichung bzw. Anwendung sowie zudem auch eine gute Verträglichkeit, verbunden mit geringen Nebenwirkungen, gegeben sein soll.

Eine wiederum weitere Aufgabe der vorliegenden Erfindung ist auch darin zu sehen, dass ein entsprechender Wirkstoff bzw. eine diesbezügliche Zusammensetzung aufgefunden bzw. bereitgestellt werden soll, welcher bzw. welche sich zur Infektionsprophylaxe bzw. zur Verringerung der viralen Last einer infizierten Person eignet.

Darüber hinaus ist eine weitere Aufgabe der vorliegenden Erfindung auch darin zu sehen, einen entsprechenden Wirkstoff bzw. eine diesbezügliche Zusammensetzung aufzufinden bzw. bereitzustellen, welcher bzw. welche im Rahmen einer (Gesamt-)Medikation mit weiteren Wirkstoffen, insbesondere antiviralen Wirkstoffen, eingesetzt werden kann, und zwar auch im Hinblick auf eine Bereitstellung einer Wirkverstärkung bzw. -ergänzung bei der Behandlung von durch Corona-Viren, vorzugsweise SARS-CoV-2, hervorgerufenen (Virus-) Erkrankungen, bevorzugt von COVID-19.

In vollkommen überraschender Weise hat die Anmelderin nunmehr herausgefunden, dass sich Octenidin und/oder dessen Salze und/oder Ester, vorzugsweise Octenidindihydrochlorid, unerwartet und in effizienter Weise als antiviraler Wirkstoff für die prophylaktische bzw. therapeutische topische (lokale) Behandlung von viralen Erkrankungen, insbesondere von durch Corona-Viren, vorzugsweise SARS-CoV-2, hervorgerufenen (Virus-)Erkrankungen, bevorzugt von COVID-19, eignet. Entsprechendes gilt gleichermaßen auch für eine hierauf basierende Zusammensetzung, insbesondere pharmazeutische Zusammensetzung, welche Octenidin und/oder dessen Salze und/oder Ester, vorzugsweise Octenidindihydrochlorid, als Wirkstoff enthält. Erfindungsgemäß wird dabei insbesondere auf eine topische (lokale) Anwendung im Mund-, Rachen- und/oder Nasenraum, insbesondere im Mund- und Rachenraum, abgestellt und zwar insbesondere auf Basis einer Lutschtablette.

Zur Lösung der zuvor geschilderten Aufgabe schlägt die vorliegende Erfindung daher - gemäß einem **ersten** Aspekt der vorliegenden Erfindung - die erfindungsgemäße Zusammensetzung, insbesondere pharmazeutische Zusammensetzung, zur Verwendung bei der prophylaktischen und/oder therapeutischen topischen (lokalen) Behandlung, insbesondere prophylaktischen und/oder therapeutischen antiviralen topischen Behandlung, von viralen Erkrankungen, nämlich von durch Corona-Viren, vorzugsweise SARS-CoV-2, hervorgerufenen (Virus-)Erkrankungen, bevorzugt von COVID-19, gemäß Patentanspruch 1 vor. Jeweils vorteilhafte Weiterbildungen und Ausgestaltungen dieses Erfindungsaspekts sind Gegenstand der betreffenden Unteransprüche sowie Nebenansprüche.

Vorliegend beschrieben - gemäß einem vorliegenden Aspekt - ist zudem die Verwendung einer Zusammensetzung, insbesondere pharmazeutischen Zusammensetzung (zur Herstellung eines Arzneimittels oder Medikaments) zur prophylaktischen bzw. therapeutischen topischen (lokalen) Behandlung von durch Corona-Viren, vorzugsweise SARS-CoV-2, hervorgerufenen (Virus-)Erkrankungen, bevorzugt von COVID-19.

Gleichermaßen ist vorliegend beschrieben - gemäß einem vorliegenden Aspekt - Octenidin bzw. dessen Salze bzw. Ester, vorzugsweise Octenidindihydrochlorid, zur Verwendung bei der prophylaktischen bzw. therapeutischen topischen (lokalen) Behandlung von viralen Erkrankungen, insbesondere von durch Corona-Viren, vorzugsweise SARS-CoV-2, hervorgerufenen (Virus-)Erkrankungen, bevorzugt von COVID-19.

Wiederum ist vorliegend beschrieben - gemäß einem vorliegenden Aspekt - zudem die Verwendung von Octenidin bzw. dessen Salze bzw. Ester, vorzugsweise Octenidindihydrochlorid, (zur Herstellung eines Arzneimittels oder Medikaments) zur prophylaktischen bzw. therapeutischen topischen (lokalen) Behandlung von viralen Erkrankungen, insbesondere von durch Corona-Viren, vorzugsweise SARS-CoV-2, hervorgerufenen (Virus-)Erkrankungen, bevorzugt von COVID-19.

Ebenfalls ist vorliegend beschrieben - gemäß einem vorliegenden Aspekt - zudem das Verfahren zur prophylaktischen bzw. therapeutischen topischen (lokalen) Behandlung von viralen Erkrankungen, insbesondere von durch Corona-Viren, vorzugsweise SARS-CoV-2, hervorgerufenen (Virus-)Erkrankungen, bevorzugt von COVID-19.

Es versteht sich von selbst, dass Ausgestaltungen, Ausführungsformen, Vorteile und dergleichen, welche nachfolgend zu Zwecken der Vermeidung von Wiederholungen nur zu einem (Erfindungs-)Aspekt aufgeführt sind, selbstverständlich auch in Bezug auf die übrigen (Erfindungs-)Aspekte entsprechend gelten, ohne dass dies einer gesonderten Erwähnung bedarf.

Weiterhin versteht es sich von selbst, dass bei nachfolgenden Angaben von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von dem angegebenen Bereich bzw. Angaben abgewichen werden kann, ohne dass der Rahmen der vorliegenden Erfindung verlassen ist.

Ferner ist bei allen nachstehend genannten relativen bzw. prozentualen, insbesondere gewichtsbezogenen Mengenangaben zu beachten, dass diese Angaben im Hinblick auf das herangezogene Bezugssystem (z. B. Darreichungsform bzw. Zusammensetzung) vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Darüber hinaus gilt für die Beschreibung der vorliegenden Erfindung, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Insbesondere gilt für die die Erfindung charakterisierenden Merkmale, dass sämtliche Kombinationsmöglichkeiten dieser Merkmale als offenbart gelten, wobei Ausführungsformen vergleichbarer bzw. entsprechender Präferenz der verschiedenen Merkmale in ihrer Kombination bevorzugt sind (z.B. Mengen bzw. Mengenbereiche der betreffenden Wirk- und Inhaltsstoffe gleicher Präferenz oder dergleichen).

Dabei gilt insbesondere auch, dass für die nachfolgend angeführten, die verschiedenen Inhaltsstoffe, insbesondere Wirkstoffe, der erfindungsgemäßen Zusammensetzung oder dergleichen betreffenden Mengenangaben, insbesondere relativen Mengenangaben oder absoluten Mengenangaben gleicher Präferenz bzw. gleicher Bevorzugungsebene, jeweilige und die verschiedenen Inhaltsstoffe, insbesondere Wirkstoffe, betreffende Kombinationen mit entsprechender Präferenz bzw. Bevorzugung mitoffenbart sind. Ebenfalls sind auch sämtliche anderweitige Kombinationen (d.h. Kombinationen auf Basis unterschiedlicher Präferenzen bzw. unterschiedlicher Bevorzugungsebenen) mitoffenbart.

Dies vorausgeschickt, wird die vorliegende Erfindung nunmehr nachfolgend im Detail erläutert:
Gegenstand der vorliegenden Erfindung - gemäß einem **ersten** Aspekt der vorliegenden Erfindung - ist somit die erfindungsgemäße Zusammensetzung, insbesondere pharmazeutische Zusammensetzung, zur Verwendung bei der prophylaktischen und/oder therapeutischen topischen (lokalen) Behandlung, insbesondere prophylaktischen und/oder therapeutischen antiviralen topischen Behandlung, von durch Corona-Viren, vorzugsweise SARS-CoV-2 hervorgerufenen Viruserkrankungen, bevorzugt von COVID-19, und zur topischen Anwendung auf die Schleimhäute des Mund-, Rachen- und/oder Nasenraums,
wobei die Zusammensetzung als antiviralen Wirkstoff Octenidin und/oder dessen Salze und/oder Ester, vorzugsweise Octenidindihydrochlorid, enthält,
wobei die Zusammensetzung als feste Dosierungsform in Form einer Lutschtablette vorliegt und/oder ausgebildet ist, wobei die Lutschtablette ein Gesamtgewicht im Bereich von 0,5 g bis 7 g, insbesondere im Bereich von 1 g bis 6 g, vorzugsweise im Bereich von 2 g bis 5 g, aufweist, enthaltend je Lutschtablette:
   - Octenidin und/oder dessen Salze und/oder Ester, vorzugsweise Octenidindihydrochlorid, in einer Menge im Bereich von 0,05 mg bis 15 mg, insbesondere im Bereich von 0,1 mg bis 12,5 mg, vorzugsweise im Bereich von 0,5 mg bis 10 mg, bevorzugt im Bereich von 0,75 mg bis 7,5 mg, besonders bevorzugt im Bereich von 1 mg bis 5 mg;
   - gegebenenfalls mindestens ein Lokalanästhetikum, insbesondere Lidocain, bevorzugt Lidocainhydrochlorid, in einer Menge im Bereich von 0,1 mg bis 50 mg, insbesondere im Bereich von 1 mg bis 20 mg, vorzugsweise im Bereich von 3 mg bis 15 mg, bevorzugt im Bereich von 4 mg bis 10 mg;
   - gegebenenfalls mindestens ein Säuerungsmittel in Form einer organischen Säure oder deren Salze oder Ester, bevorzugt Weinsäure und/oder Citronensäure, in einer Menge im Bereich von 0,5 mg bis 100 mg, insbesondere im Bereich von 1 mg bis 50 mg, vorzugsweise im Bereich von 2 mg bis 20 mg;
   - gegebenenfalls mindestens ein Anisöl, insbesondere Sternanisöl, in einer Menge im Bereich von 0,2 mg bis 80 mg, insbesondere im Bereich von 0,5 mg bis 40 mg, vorzugsweise im Bereich von 0,75 mg bis 15 mg;
   - gegebenenfalls mindestens ein ätherisches Öl, insbesondere Pfefferminzöl, in einer Menge im Bereich von 0,01 mg bis 5 mg, insbesondere im Bereich von 0,05 mg bis 3 mg, vorzugsweise im Bereich von 0,1 mg bis 1 mg;
   - gegebenenfalls mindestens ein Kaffee-Extrakt und/oder Tee-Extrakt, insbesondere Kaffee-Extrakt, in einer Menge im Bereich von 0,05 mg bis 100 mg, insbesondere im Bereich von 0,5 mg bis 50 mg, vorzugsweise im Bereich von 1 mg bis 20 mg;
   - gegebenenfalls Propylenglykol in einer Menge im Bereich von 0,05 mg bis 50 mg, insbesondere im Bereich von 0,5 mg bis 20 mg, vorzugsweise im Bereich von 1 mg bis 10 mg;
   - gegebenenfalls mindestens eine Schleimdroge und/oder deren Extrakt in einer Menge im Bereich von 0,5 mg bis 200 mg, insbesondere im Bereich von 1 mg bis 100 mg, vorzugsweise im Bereich von 5 mg bis 50 mg;
   - gegebenenfalls mindestens einen weiteren Wirk- und/oder Inhaltsstoff, insbesondere pharmazeutischen Zusatz- und/oder Hilfsstoff, insbesondere ausgewählt aus der Gruppe von Verarbeitungshilfsstoffen, Stabilisatoren, Emulgatoren, Antioxidantien, Konservierungsstoffen, Feuchthaltemitteln, Süßstoffen und Süßungsmitteln, pH-Stellmitteln, pH-Puffersubstanzen, Verdickungsmitteln, Aromastoffen, Antiseptika, Färbe-, Puffer-, Riech-, Duft-, Streck-, Binde-, Netz- und/oder Konservierungsstoffen sowie deren Kombinationen und Mischungen, in einer Menge im Bereich von 0,1 mg bis 500 mg, insbesondere im Bereich von 0,5 mg bis 250 mg, vorzugsweise im Bereich von 1 mg bis 100 mg;
   - mindestens einen Zucker und/oder mindestens einen Zuckeraustauschstoff als Exzipient; wobei der Zucker ausgewählt ist aus der Gruppe von Saccharose, Glucose, insbesondere Dextrose, und Fructose und/oder wobei der Zuckeraustauschstoff ausgewählt ist aus Zuckeralkoholen, vorzugsweise aus der Gruppe von Mannit, Xylit, Sorbit, Isomalt, Maltitsirup, Lactit, Leucrose, Fructooligosacchariden, Glucanen, Polyglucose sowie deren Kombinationen und Mischungen, besonders bevorzugt Isomalt und/oder Mannit sowie deren Kombinationen und Mischungen, ganz besonders bevorzugt Isomalt; und in Mengen zur Bereitstellung und/oder Erhalt des Gesamtgewichts der Lutschtablette,
wobei die Lutschtablette zumindest im Wesentlichen frei ist von Phenoxyethanol.

Denn - wie auch zuvor ausgeführt - die Anmelderin hat in vollkommen überraschender Weise gefunden, dass sich Octenidin und/oder dessen Salze und/oder Ester, vorzugsweise Octenidindihydrochlorid, unerwartet und in effizienter Weise als Wirkstoff zur topischen (lokalen) Behandlung von viralen Erkrankungen, nämlich von durch Corona-Viren, vorzugweise SARS-CoV-2, hervorgerufenen Viruserkrankungen (d. h. viralen Infektionen bzw. Corona(virus)infektionen), bevorzugt von COVID-19, eignet.

Die von der Anmelderin im Rahmen der vorliegenden Erfindung überraschend aufgefundene, spezielle und neue sowie erfinderische Anwendung bzw. medizinische Indikation für Octenidin bzw. dessen Salze und/oder Ester, vorzugsweise Octenidindihydrochlorid, ist im Stand der Technik bislang nicht beschrieben und auch nicht angedacht oder erkannt worden, obwohl es sich bei Octenidin bzw. dessen Salze bzw. Ester, vorzugsweise Octenidindihydrochlorid, grundsätzlich um einen an sich bekannten Wirkstoff handelt.

Wie nachfolgend noch im Detail ausgeführt, liegt die erfindungsgemäße Zusammensetzung in einer festen Dosierungsform zum Lutschen, nämlich in Form einer Lutschtablette, vor, wobei die Zusammensetzung nach der Erfindung eine therapeutisch wirksame Menge an Octenidin bzw. dessen Salze und/oder Ester, vorzugsweise Octenidindihydrochlorid, enthält.

Im Rahmen der vorliegenden Erfindung bezieht sich der Begriff "topische Behandlung" bzw. "topische Anwendung" insbesondere auf die lokale prophylaktische bzw. therapeutische Behandlung der in Rede stehenden Viruserkrankung bzw. auf die Anwendung der Zusammensetzung im Mund-, Rachen- und/oder Nasenraum, vorzugsweise Mund- und/oder Rachenraum. Insbesondere bezieht sich der vorgenannte Begriff auf die Schleimhäute des Mund-, Rachen- und/oder Nasenraums, insbesondere Schleimhäute des Mund- und/oder Rachenraums. In diesem Zusammenhang hat die Anmelderin völlig überraschend gefunden, dass Octenidin und/oder dessen Salze und/oder Ester, vorzugsweise Octenidindihydrochlorid, bei derartiger topischer bzw. lokaler Anwendung eine antivirale Wirksamkeit aufweist, und zwar insbesondere gegenüber Corona-Viren, vorzugsweise SARS-CoV-2.

Die Anmelderin hat in diesem Zusammenhang überraschenderweise festgestellt, dass sich Octenidin bzw. dessen Salze und/oder Ester, vorzugsweise Octenidindihydrochlorid, ausgezeichnet zur gezielten topischen Anwendung im Mund-, Rachen- und/oder Nasenraum, insbesondere Mund- und Rachenraum, bzw. zur topischen Anwendung auf Schleimhäute des Mund-, Rachen- und/oder Nasenraums, insbesondere des Mund- und Rachenraums, eignet, und zwar im Hinblick auf die prophylaktische bzw. therapeutische topische Behandlung von viralen Erkrankungen, welche durch Corona-Viren, vorzugsweise SARS-CoV-2, nämlich Covid-19, hervorgerufen werden.

In diesem Zusammenhang führt die ziel- und zweckgerichtet topische bzw. lokale Anwendung von Octenidin bzw. dessen Salze bzw. Ester, vorzugsweise Octenidindihydrochlorid, wie es seitens der Anmelderin gezeigt werden konnte, zu einer deutlichen Verringerung der Virenlast im Mund-, Rachen- und/oder Nasenraum, so dass auf dieser Basis auch mit der Erkrankung einhergehende Symptome gelindert werden können.

Auf dieser Basis kann entsprechend auch der symptomatische Verlauf der Erkrankung für infizierte Personen positiv beeinflusst werden, einhergehend mit der Verringerung zugrundeliegender Symptome bzw. Beschwerden. Dies wirkt sowohl einer systemischen Ausbreitung des Virus im Körper einer infizierten Person als auch einer Weitergabe bzw. Abgabe des Virus in die Umwelt entgegen, so dass für weitere bzw. dritte Personen ein geringeres Infektionsrisiko vorliegt.

Die erfindungsgemäße Zusammensetzung wirkt, veranschaulicht gesprochen, insbesondere bereits an der Eintrittspforte für das Virus, nämlich im Mund-, Rachen- bzw. Nasenraum, so dass die Gefahr einer Infektion selbst gemindert wird und zudem die Gefahr eines systemischen Übergriffs bzw. einer systemischen Ausbreitung des Virus verringert wird.

Erfindungsgemäß ist es auch völlig überraschend, dass die Zusammensetzung nach der Erfindung auf Basis von Octenidin bzw. dessen Salze bzw. Ester beispielsweise auch im Rahmen einer Infektions- bzw. Expositionsprophylaxe eingesetzt werden kann. Denn infolge der antiviralen Wirkung von Octenidin bzw. dessen Salze bzw. Ester, vorzugsweise Octenidindihydrochlorid, kann die Virenlast im Mund-, Rachen- und/oder Nasenraum effektiv verringert werden, was mit einem verringerten Risiko der (Eigen-)Infektion und darüber hinaus auch der Infektion weiterer bzw. dritter Personen einhergeht. In diesem Zusammenhang kann die Zusammensetzung nach der Erfindung in überraschender Weise somit zur Infektionsprophylaxe sowie zur Expositionsprophylaxe, insbesondere zur Post- bzw. Präexpositionsprophylaxe, eingesetzt werden, wie auch nachfolgend noch angeführt.

Darüber hinaus eignet sich die erfindungsgemäße Zusammensetzung auch zur Anwendung im Rahmen einer Co-Medikation mit weiteren antiviralen Wirkstoffen oder dergleichen, und zwar auch mit systemischen Wirkstoffen. Insbesondere kann die erfindungsgemäße Zusammensetzung zur begleiteten bzw. ergänzenden und/oder unterstützenden Behandlung der zugrundeliegenden viralen Erkrankungen, insbesondere COVID-19, eingesetzt werden. Insbesondere kann die erfindungsgemäße Zusammensetzung auch in entsprechende Behandlungs- bzw. Therapiekonzepte eingebunden werden.

Zudem weist die erfindungsgemäße Zusammensetzung mit der topischen Anwendung im Mund-, Rachen- bzw. Nasenraum eine einfache Handhabung bzw. gute Anwendungseigenschaften auf. Zudem liegt insbesondere auch infolge der lokalen bzw. topischen Verabreichung des Wirkstoffs insgesamt eine gute Verträglichkeit der Zusammensetzung nach der Erfindung bei gleichzeitig hoher Wirkeffizienz vor.

Die vorliegende Erfindung beruht auf einer völlig überraschend gefundenen insbesondere lokalen Einflussnahme (nämlich im Mund-, Rachen- bzw. Nasenraum) von Octenidin bzw. dessen Salze bzw. Ester, vorzugsweise Octenidindihydrochlorid, insbesondere auf Corona-Viren, wie SARS-CoV-2, und zwar insbesondere infolge eines lokalen antiviralen Wirkeffekts, was einen wertvollen Beitrag zum Schutz insbesondere vor SARS-CoV-2 darstellt (und zwar was die Behandlung von COVID-19 bzw. eine effiziente Infektions- bzw. Expositionsprophylaxe sowie die Verringerung der Gefahr eines systemischen Übergriffs des Virus anbelangt).

Der Begriff "pharmazeutische Zusammensetzung", wie er im Rahmen der vorliegenden Erfindung verwendet wird, ist dabei sehr breit zu verstehen und umfasst nicht nur pharmazeutische Präparate bzw. Pharmazeutika und Arzneimittel als solche, sondern auch sogenannte Medizinprodukte, Lebensmittel oder Nahrungsergänzungsmittel.

Der Begriff "antiviral", wie er erfindungsgemäß für Octenidin bzw. dessen Salzen bzw. Estern, vorzugsweise Octenidindihydrochlorid, bzw. die erfindungsgemäße Zusammensetzung sowie im Hinblick auf die zugrundeliegende topische Behandlung der viralen Erkrankungen verwendet wird, ist im Rahmen der vorliegenden Erfindung sehr breit zu verstehen und bezieht sich insbesondere auf eine gegen die zugrundeliegenden Viren, nämlich Corona-Viren, vorzugsweise SARS-CoV-2, gerichtete Wirksamkeit. Die erfindungsgemäß in Bezug auf Octenidin bzw. dessen Salze bzw. Ester, vorzugsweise Octenidindihydrochlorid, angeführte antivirale Wirksamkeit kann sich beispielsweise und in nicht beschränkender Weise auf eine Inaktivierung von Viren bzw. Viruspartikeln, insbesondere auch außerhalb von deren Wirtszellen, bzw. auf ein "Abtöten" der Viren beziehen. Gleichermaßen kann sich die antivirale Wirksamkeit beispielsweise auch auf eine Verhinderung bzw. Verringerung der Vermehrung bzw. der Entstehung der Viren bzw. Viruspartikeln insbesondere in Wirtszellen bzw. auf eine Verhinderung bzw. Verringerung der Freisetzung von Viren bzw. Viruspartikeln aus Wirtszellen beziehen. Zudem kann sich die antivirale Wirksamkeit in diesem Zusammenhang und in gleichermaßen nicht beschränkender Weise auf eine Unterbindung bzw. Verringerung des (viralen) Replikationszyklus beziehen. Zudem kann sich die antivirale Wirkung auch auf die Ausbildung einer Barrierefunktion ("Schutzfilm") beziehen, so dass hierdurch das Eindringen von Viren in Wirtszellen erschwert wird. Im Allgemeinen und zusammenfassend bezieht sich somit der Begriff "antiviral", wie er erfindungsgemäß verwendet wird, beispielhaft und ohne sich auf die zugrundeliegenden Mechanismen berufen oder beschränken zu wollen, auf eine Inaktivierung bzw. "Abtöten" der Viren, Unterbindung bzw. Verringerung der Vermehrung bzw. Replikation der Viren, Ausbildung eines Schutzfilms bzw. einer Barrierefunktion gegenüber den Viren oder dergleichen.

Die Begriffe "Salze" bzw. "Ester", wie sie vorliegend im Hinblick auf die angeführten Wirk- bzw. Inhaltsstoffe angeführt werden, beziehen sich insbesondere auf die jeweiligen pharmazeutisch verträglichen bzw. unbedenklichen Salze bzw. Ester der angeführten Wirk- bzw. Inhaltsstoffe.

Auf Basis der erfindungsgemäßen Konzeption kann somit die Virusvermehrung bzw. Virenlast auch innerhalb der ersten Tage nach einer Infektion verringert bzw. unterbunden werden, was auch mit einer Verringerung bzw. Vermeidung des Auftretens von krankheitsspezifischen Symptomen einhergeht und dem Auftretens schwerer Krankheitsverläufe bzw. einer systemischen Überprüfung entgegenwirkt. Zudem kann infolge der Verringerung der Virenlast im Mund-, Rachen- bzw. Nasenraum insbesondere auch eine lokal auftretende Krankheitssymptomatik reduziert bzw. unterbunden werden. Zudem kann die Wahrscheinlichkeit einer Infektion nach erfolgter Exposition verringert und die Weitergabe des Virus an weitere bzw. dritte Personen verhindert bzw. reduziert werden.

Im Rahmen der vorliegenden Erfindung ist es dabei gleichermaßen überraschend, dass die spezielle topische Applikation der Zusammensetzung nach der Erfindung, insbesondere die topische Anwendung im Mund-, Rachen- bzw. Nasenraum, vorzugsweise im Mund- und Rachenraum, mit einer hohen Effizienz und Wirksamkeit gegenüber den zugrundeliegenden Viren, nämlich Corona-Viren, vorzugsweise SARS-CoV-2, einhergeht. In diesem Zusammenhang kommt auch der speziellen Darreichungsform der erfindungsgemäßen Zusammensetzung bzw. der zugrundeliegenden Galenik eine große Bedeutung zu, wonach die erfindungsgemäße Zusammensetzung in einer festen Darreichungsform, nämlich einer Lutschtablette, vorliegt bzw. verabreicht wird, wie nachfolgend noch im Detail dargelegt. Zudem wird durch die spezielle Dosierungsform, nämlich feste Dosierungsform auf Lutschtablettenbasis, eine definierte und zeitlich optimierte Freisetzung der Wirksubstanz insbesondere im Mund- und Rachenraum erreicht, was auch zu einer nochmals verbesserten antiviralen Wirkung gegenüber den in Rede stehenden Viren führt.

Das pharmazeutische Wirkpotential von Octenidin bzw. Octenidindihydrochlorid in Bezug auf die topische (lokale) Behandlung von viralen Erkrankungen, und zwar von durch Corona-Viren, vorzugsweise SARS-CoV-2, hervorgerufenen Viruserkrankungen, bevorzugt von COVID-19, mit der diesbezüglichen Anwendung im Mund-, Rachen- bzw. Nasenraum, vorzugsweise im Mund- und Rachenraum, und zwar insbesondere auch in fester Dosierungsform zum Lutschen, ist bislang unerkannt geblieben. Diese Erkenntnis geht erst auf die Anmelderin zurück. Denn erst die Anmelderin hat den Wirkstoff Octenidin bzw. Octenidindihydrochlorid einer speziellen topischen Anwendung im Mund-, Rachen- bzw. Nasenraum, vorzugsweise im Mund- und Rachenraum, zur gezielten prophylaktischen bzw. therapeutischen antiviralen topischen Behandlung der erfindungsgemäß angeführten viralen Erkrankungen zugeführt, und zwar auf Basis bzw. in Form einer festen galenischen Zubereitung zum Lutschen, nämlich in Form einer Lutschtablette. Auf diese Weise kommt das Wirkpotential von Octenidin bzw. Octenidindihydrochlorid in Bezug auf seine spezielle antivirale Wirkung gegenüber Corona-Viren und insbesondere SARS-CoV-2 im besonderen Maße zur Geltung bzw. zum Einsatz, einhergehend mit der hohen Wirksamkeit im Hinblick auf die prophylaktische bzw. therapeutische antivirale topische Behandlung der zugrundeliegenden viralen Erkrankungen, insbesondere im Hinblick auf COVID-19.

Der erfindungsgemäß eingesetzte Wirkstoff Octenidin (CAS-Nr.: 71251-02-0) bzw. Octenidindihydrochlorid (CAS-Nr.: 70775-75-6) gehört zu der Gruppe der quartären Ammoniumverbindungen bzw. zu der chemischen Gruppe der Bipyridine. Octenidin bzw. Octenidindihydrochlorid weist in diesem Zusammenhang zwei kationenaktive Zentren auf. Octenidindihydrochlorid ist der internationale Freiname für 1,1'-(1,10-Decandiyl)bis[4-(octylamino)pyridinium]-dichlorid bzw. für 1,1'-Decamethylen[(1,4-dihydro-4-octylimimo)pyridinium]dichlorid.

Für weitergehende Einzelheiten zu Octenidin bzw. dessen Salzen bzw. Estern bzw. Octenidindihydrochlorid kann beispielsweise verwiesen werden auf Römpp Chemielexikon, 10. Auflage, Band 4, 1998, Georg-Thieme-Verlag, Stuttgart/New York, Seite 2986, Stichwort: "Octenidindihydrochlorid", wobei der gesamte Offenbarungsgehalt, einschließlich der dort genannten Literatur, hiermit durch Bezugnahme vollumfänglich eingeschlossen ist.

Gemäß einer erfindungsgemäß bevorzugten Ausführungsform kann die Zusammensetzung nach der Erfindung das Octenidin und/oder dessen Salze und/oder Ester, wie zuvor angeführt, in Form eines Hydrochloridsalzes, vorzugsweise Octenidindihydrochlorid, enthalten. Erfindungsgemäß verhält es sich gemäß einer bevorzugten Ausführungsform insbesondere derart, dass das Octenidin und/oder dessen Salze und/oder Ester ein Octenidin-Hydrochloridsalz, vorzugsweise Octenidindihydrochlorid, ist.

In besonders bevorzugter Weise enthält die Zusammensetzung nach der Erfindung als antiviralen Wirkstoff Octenidindihydrochlorid. Die Verwendung von Octenidindihydrochlorid geht mit einer besonders hohen Wirksamkeit und definiertem Wirkspektrum der Zusammensetzung nach der Erfindung einher.

Erfindungsgemäß verhält es sich dabei insbesondere derart, dass die Zusammensetzung nach der Erfindung das Octenidin und/oder dessen Salze und/oder Ester, vorzugsweise Octenidindihydrochlorid, in pharmazeutisch und/oder therapeutisch wirksamen, insbesondere in pharmazeutisch und/oder therapeutisch antiviral wirksamen Mengen, enthält.

Die Wirksamkeit der erfindungsgemäßen Zusammensetzung kann noch dadurch gesteigert bzw. maßgeschneidert werden, dass der Zusammensetzung mindestens ein Lokalanästhetikum zugesetzt wird. Erfindungsgemäß kann es somit vorgesehen sein, dass die Zusammensetzung nach der Erfindung mindestens ein Lokalanästhetikum enthält. Hierdurch kann beispielsweise eine mit der Viruserkrankung einhergehende lokale Schmerzsymptomatik, insbesondere im Mund-, Rachen- bzw. Nasenraum, bevorzugt im Mund- und Rachenraum, gezielt behandelt werden. Hierdurch wird auch, beispielsweise infolge eines verringerten Räusperns oder eines normalisierten Schluckverhaltens, das betroffene Gewebe geschont und das Einwirkverhalten von Octenidin bzw. Octenidindihydrochlorid unterstützt.

Was das erfindungsgemäß einsetzbare Lokalanästhetikum anbelangt, so können grundsätzlich sämtliche zur topischen Anwendung geeigneten Lokalanästhetika zur Anwendung kommen. Für diesbezügliche Einzelheiten zu Lokalanästhetika kann beispielsweise verwiesen werden auf E. Mutschler et al., "Mutschler Arzneimittelwirkungen - Lehrbuch der Pharmakologie und Toxikologie", 8. Auflage, Wissenschaftliche Verlagsgesellschaft mbH, Stuttgart, 2001, Seiten 267 ff., und Römpp Chemielexikon, 10. Auflage, Band 3, Georg Thieme Verlag, Stuttgart/New York, 1997, Seiten 2442, Stichwort: "Lokalanästhetika" sowie die dort jeweils referierte Literatur.

Erfindungsgemäß kann das Lokalanästhetikum insbesondere ein Lokalanästhetikum auf Basis eines organischen Säureesters oder Säureamids, vorzugsweise Säureamids, sein. In diesem Zusammenhang kann das Lokalanästhetikum ausgewählt sein aus der Gruppe von Lokalanästhetika vom Estertyp und Lokalanästhetika vom Amidtyp sowie deren Kombinationen und Mischungen, vorzugsweise Lokalanästhetika vom Amidtyp.

Erfindungsgemäß kann das Lokalanästhetikum ausgewählt sein aus der Gruppe von Benzocain, Procain, Tetracain, Lidocain, Etidocain, Prilocain, Mepivacain, Bupivacain und S-Ropivacin und deren Salzen und Estern sowie deren Kombinationen und Mischungen, insbesondere Lidocain und dessen Salzen und Estern, vorzugsweise Lidocainhydrochlorid.

Gemäß einer erfindungsgemäß bevorzugten Ausführungsform kann das Lokalanästhetikum ausgewählt sein aus der Gruppe von Lidocain (2-Diethylamino-N-(2,6-dimethylphenyl)acetamid) und dessen Salzen und Estern, bevorzugt Lidocainhydrochlorid.

Erfindungsgemäß kann es insbesondere vorgesehen sein, dass die Zusammensetzung, insbesondere als Lokalanästhetikum, Lidocain (2-Diethylamino-N-(2,6-dimethylphenyl)acetamid) und/oder dessen Salze und/oder Ester, bevorzugt Lidocainhydrochlorid, enthält.

Bei Lidocain handelt es sich um ein Lokalanästhetikum vom Amidtyp, insbesondere vom Aminoamidtyp, welches eine gute lokale Wirksamkeit bei gleichzeitig schnellem Wirkeintritt und guter Verträglichkeit aufweist. Lidocain blockiert reversibel spannungsabhängige Natriumkanäle in den Zellmembranen von Nervenzellen. Insbesondere hemmt Lidocain den Einstrom von Natriumionen über spannungsabhängige Natriumkanäle in die Nervenzellen, was zu einer verminderten Erregbarkeit von Nervenfasern führt, da der zur Ausbildung eines Aktionspotentials erforderliche Anstieg der Natriumpermeabilität verringert ist. Hierdurch wird das Schmerzempfinden herabgesetzt.

Insbesondere kann das Lokalanästhetikum ausgewählt sein aus der Gruppe von Lidocain sowie dessen pharmazeutisch verträglichen Salzen und Estern. Gemäß einer erfindungsgemäß bevorzugten Ausführungsform wird das Lokalanästhetikum in Form von Lidocainhydrochlorid eingesetzt. Lidocainhydrochlorid weist im Vergleich zu Lidocain insbesondere eine höhere Wasserlöslichkeit auf. Hierdurch wird - ohne sich auf diese Theorie berufen oder beschränken zu wollen - die Wirkstofffreisetzung bei Kontakt mit Flüssigkeit bzw. Speichel nochmals verbessert, was auch zu verbesserten Wirkeigenschaften führt. Hinzu kommt, dass
- gleichermaßen ohne sich auf diese Theorie berufen oder beschränken zu wollen - das mit den vorliegenden (Virus-)Erkrankungen insbesondere im Mund/Rachen-Raum vorliegende entzündete Gewebe, insbesondere infolge einer lokalen Lactatacidose, einen niedrigeren pH-Wert gegenüber normalen bzw. nichtentzündetem Gewebe aufweist. Auch von daher weist Lidocainhydrochlorid ein gutes Penetrationsvermögen auf, da diesbezüglich bereits die protonierte Form vorliegt.

Erfindungsgemäß kann die Zusammensetzung das Lokalanästhetikum, insbesondere Lidocain und/oder dessen Salze und/oder Ester, vorzugsweise Lidocainhydrochlorid, in pharmazeutisch und/oder therapeutisch wirksamen, insbesondere in pharmazeutisch und/oder therapeutisch lokalanästhetisch wirksamen Mengen, enthalten.

Für weitere Ausführungen zu Lidocain kann beispielsweise auf RÖMPP Chemielexikon, 10. Auflage, Band. 3, 1997, Georg Thieme-Verlag, Stuttgart/New York, Stichwort: "Lidocain" sowie auf die dort jeweils in Bezug genommene Literatur verwiesen werden, deren gesamter Inhalt hiermit durch Bezugnahme vollumfänglich eingeschlossen ist. Zudem kann verwiesen werden auf die Ausführungen in *Lidocaine Hydrochloride, Europäische Pharmakopöe (European Pharmacopoeia) (Ph. Eur.), 9. Auflage (Januar 2017).* Zudem kann in Bezug auf Lidocain auf die Ausführungen in *European Pharmacopoeia 8.0, European Directorate for the Quality of Medicines and Healthcare,* Seiten 2620/2621, Stichwort: "Lidocaine" verwiesen werden. Darüber hinaus kann in Bezug auf Lidocainhydrochlorid auf *European Pharmacopoeia 8.0, European Directorate for the Quality of Medicines and Healthcare,* Seiten 2620/2621, Stichwort: "Lidocaine Hydrochloride" verwiesen werden.

Die erfindungsgemäße Zusammensetzung kann darüber hinaus auch die nachfolgend angeführten Wirk- bzw. Inhaltsstoff aufweisen:
Gemäß einer erfindungsgemäßen Ausführungsform kann die Zusammensetzung nach der Erfindung mindestens ein Antiphlogistikum, insbesondere Benzydamin und/oder dessen Salze und/oder Ester, bevorzugt Benzydaminhydrochlorid, enthalten.

Insbesondere kann die Zusammensetzung das Antiphlogistikum, insbesondere Benzydamin und/oder dessen Salze und/oder Ester, bevorzugt Benzydaminhydrochlorid, in pharmazeutisch und/oder therapeutisch wirksamen, insbesondere in pharmazeutisch und/oder therapeutisch antiphlogistisch wirksamen Mengen, enthalten.

Bei Benzydamin handelt es sich insbesondere um ein benzyliertes Indazol-Derivat aus der Wirkstoffgruppe der Antiphlogistika. Benzydamin weist insbesondere entzündungshemmende, schmerzlindernde und leicht antimikrobielle Wirkeigenschaften auf. Einhergehend mit der Verwendung von Benzydamin können im Rahmen der Anwendung der erfindungsgemäßen Zusammensetzung insbesondere Schmerzen und Reizungen im Mund- und Rachenraum weiterführend und im Zusammenspiel mit den weiteren Wirk- bzw. Inhaltsstoffen symptomatisch behandelt werden. Auch von daher können die mit der Infektion einhergehenden Krankheitssymptome weiterführend gelindert werden, zumal Benzydamin zusätzlich auch fiebersenkend ist.

Gemäß einer weiteren Ausführungsform der vorliegenden Erfindung kann es weiterhin vorgesehen sein, dass die Zusammensetzung nach der Erfindung mindestens ein nichtsteroidales Antiphlogistikum (NSAR-Wirkstoff) enthält. Diesbezüglich kann das nichtsteroidale Antiphlogistikum (NSAR-Wirkstoff) ausgewählt sein aus der Gruppe von Flurbiprofen, Ibuprofen, Dexibuprofen, Naproxen, Ketoprofen, Dexketoprofen, Tiaprofensäure, Diclofenac und Acetylsalicylsäure und deren Salzen und Estern sowie deren Kombinationen und Mischungen, insbesondere Flurbiprofen und dessen Salzen und Estern, vorzugsweise Flurbiprofen. Insbesondere kann es erfindungsgemäß vorgesehen sein, dass die Zusammensetzung Flurbiprofen und/oder dessen Salze und/oder Ester, bevorzugt Flurbiprofen, enthält.

Weiterhin kann die Zusammensetzung das nichtsteroidale Antiphlogistikum (NSAR-Wirkstoff), insbesondere Flurbiprofen und/oder dessen Salze und/oder Ester, bevorzugt Flurbiprofen, in pharmazeutisch und/oder therapeutisch wirksamen, insbesondere in pharmazeutisch und/oder therapeutisch nichtsteroidalantiphlogistisch wirksamen Mengen, enthalten.

Flurbiprofen, welches im Rahmen der vorliegenden Erfindung in bevorzugter Weise als nichtsteroidales Antiphlogistikum eingesetzt wird, wird im Allgemeinen zu der Gruppe der Phenylalkansäurederivate der nichtsteroidalen Antirheumatika bzw. Antiphlogistika gezählt. Flurbiprofen wird ein entzündungshemmendes, schmerzlinderndes und abschwellendes Wirkprofil zugesprochen. Insbesondere kann gezielt eine lokale Behandlung von Halsschmerzen bzw. Schmerzen im Mund- und Rachenraum erfolgen, und zwar gleichermaßen im Zusammenspiel mit den weiteren Wirk- und Inhaltsstoffen der erfindungsgemäßen Zusammensetzung.

Insbesondere kann die erfindungsgemäße Zusammensetzung mindestens ein Säuerungsmittel, insbesondere in Form einer organischen Säure und/oder deren Salze und/oder Ester, bevorzugt Weinsäure und/oder Citronensäure, bevorzugt Weinsäure, enthalten.

Durch die Verwendung eines Säuerungsmittels können auch die organoleptischen Eigenschaften der erfindungsgemäßen Zusammensetzung weiterführend verbessert bzw. eingestellt werden. Das Säuerungsmittel kann bei Anwendung bzw. Verabreichung der Zusammensetzung, insbesondere auf Basis einer festen Dosierungsform, wie eine Lutschtablette, auch gezielt eine Induktion bzw. Steigerung des Speichelflusses bzw. der Speichelproduktion bewirken, wodurch die Wirkstofffreisetzung positiv beeinflusst wird.

Weiterhin kann die Zusammensetzung mindestens ein Anisöl, insbesondere Sternanisöl, enthalten.

Sternanisöl kann im Allgemeinen aus dem Echten Sternanis, botanisch *Illicium verum*, gewonnen werden, wobei hierzu insbesondere eine Extraktion aus den reifen Früchten der Pflanze vorgenommen werden kann. Sternanisöl enthält als Hauptkomponente trans-Anethol, insbesondere in einer Menge von 80 Gew.-% bis 90 Gew.-%, bezogen auf das ätherische Öl. Zudem kann Sternanisöl Estragol, Foeniculin, Limonen und/oder Cineol enthalten. Für weitergehende Einzelheiten zu Sternanisöl kann verwiesen werden auf RÖMPP Lexikon Naturstoffe, erste Auflage, Georg Thieme Verlag, Stuttgart/New York, 1997, Seite 609, Stichwort: "Sternanis" und die dort referierte Literatur, deren gesamter Inhalt hiermit durch Bezugnahme eingeschlossen ist.

Anisöl, insbesondere Sternanisöl kann insbesondere zur Einstellung des Geschmacks der Zusammensetzung verwendet werden. Zudem kommt Anisöl bzw. Sternanisöl auch eine schleimlösende sowie krampflösende Eigenschaft zu.

Weiterhin kann die Zusammensetzung mindestens ein ätherisches Öl, insbesondere Pfefferminzöl, enthalten.

Das Pfefferminzöl kann insbesondere aus den oberirdischen Teilen der Pfefferminze, botanisch *Mentha piperita*, insbesondere aus den Blättern, gewonnen werden. Erfindungsgemäß eingesetztes Pfefferminzöl kann insbesondere Menthol, vorzugsweise in Mengen von 25 Gew.-% bis 45 Gew.-%, Menthon, vorzugsweise in Mengen von 20 Gew.-% bis 30 Gew.-%, und/oder Menthylacetat, vorzugsweise in Mengen von 2 Gew.-% bis 10 Gew.-%, aufweisen, jeweils bezogen auf das ätherische Öl. Für weitergehende Einzelheiten zu dem erfindungsgemäß einsetzbaren Pfefferminzöl kann zudem verwiesen werden auf RÖMPP Lexikon Naturstoffe, erste Auflage, Georg Thieme Verlag, Stuttgart/New York, 1997, Seiten 478 und 479, Stichwort: "Pfefferminzöl" und die dort referierte Literatur, deren gesamter Inhalt hiermit durch Bezugnahme eingeschlossen ist.

Durch die Verwendung eines ätherischen Öls, insbesondere Pfefferminzöl, kann der Geschmack der erfindungsgemäßen Zusammensetzung weiterführend eingestellt werden. Zudem weisen die ätherischen Öle bzw. Pfefferminzöl eine antibakterielle bzw. antifugale Wirksam auf, was im Hinblick auf die zugrundeliegende topische Anwendung von weiterem Vorteil ist, beispielsweise im Hinblick auf Sekundärinfektionen oder dergleichen.

Darüber hinaus kann die Zusammensetzung mindestens einen Kaffee-Extrakt und/oder Tee-Extrakt, insbesondere Kaffee-Extrakt, enthalten. Beispielweise können Trockenextrakte bzw. wässrige Extrakte eingesetzt werden.

Im Allgemeinen kann die erfindungsgemäße Zusammensetzung darüber hinaus auch Polypropylenglykol enthalten.

Polypropylen führt im Rahmen der Verabreichung der erfindungsgemäßen Zusammensetzung im Mund-, Rachen- bzw. Nasenraum zu einer erhöhten mucoadhäsiven Wirkung und somit zu einer längeren Kontaktzeit der Zusammensetzung mit dem Wirkstoff gegenüber dem zugrundeliegende Gewebe bzw. den Schleimhäuten. Dies geht mit einer verlängerten Einwirkzeit auch der Wirksubstanzen einher.

Darüber hinaus kann die Zusammensetzung nach der Erfindung mindestens eine Schleimdroge bzw. deren Extrakt enthalten.

Im Allgemeinen kann die Schleimdroge bzw. deren Extrakt im Rahmen der vorliegenden Erfindung ausgewählt sein aus der Gruppe von Isländisch Moos (*Lichen islandicus*), Eibisch (*Althaea officinalis L.*), Spitzwegerich (*Plantago lanceolata L.*), Malve (*Malva sylvestris L.* und M. *neglecta WALLR.* und andere), Boxhornklee (*Trigonella foenum-graecum L.*), Salep und Quitte (*Cydonia oblonga MILL.*) sowie deren Kombinationen und Mischungen, bevorzugt Isländisch Moos (*Lichen islandicus*) und/ oder Eibisch (*Althaea officinalis L.*)*.*

Erfindungsgemäß kann die Schleimdroge in Form der Droge, insbesondere in Form zerkleinerter oder pulverisierter Pflanzenteile oder Pflanzenbestandteile, eingesetzt sein und/oder vorliegen. Darüber hinaus kann die Schleimdroge in Form eines Extraktes, insbesondere in Form eines Trockenextraktes, eingesetzt sein und/oder vorliegen. Was die erfindungsgemäß gegebenenfalls eingesetzte Schleimdroge somit anbelangt, so kann diese erfindungsgemäß in Form der Droge selbst, insbesondere in Form zerkleinerter oder pulverisierter Pflanzenteile oder Pflanzenbestandteile, der Zusammensetzung zugesetzt sein. Weiterhin kann die Schleimdroge in Form eines Extrakts, insbesondere Trockenextrakts, welcher beispielsweise erhältlich ist ausgehend von einem wässrigen, alkoholischen oder wässrig-alkoholischen Extrakts der Droge, eingesetzt werden. Die Verwendung eines Extrakts einer Schleimdroge bietet gegenüber der Verwendung der Droge den Vorteil, dass hohe Konzentrationen der Schleimdroge zur Anwendung kommen, so dass auch von daher eine besonders gute Wirkung erzielt wird.

Schleimdrogen bzw. deren Extrakte wirken insbesondere reizmildernd und abdeckend. Zudem führen sie zu einer Verringerung des Hustenreizes (Antitussiva).

Die jeweiligen Schleimdrogen bzw. deren Extrakte als solche sind dem Fachmann bekannt. Für weitere Einzelheiten zu Schleimdrogen und ihren Zubereitungen, Wirkungen und Anwendungen kann verwiesen werden auf H. Wagner "Pharmazeutische Biologie - Drogen und ihre Inhaltsstoffe", Gustav Fischer Verlag, Stuttgart/New York, 1985, insbesondere Seiten 280 ff.

Erfindungsgemäß kann die Zusammensetzung weiterhin mindestens einen Exzipienten (Träger), vorzugsweise pharmakologisch und/oder physiologisch unbedenklichen Exzipienten (Träger), enthalten. Hierdurch kann die erfindungsgemäße Zusammensetzung weiterführend auch hinsichtlich ihrer Galenik eingestellt bzw. maßgeschneidert werden. Auf dieser Basis kann auch die Wirkstofffreisetzung bzw. Wirkstoffverfügbarkeit gezielt eingestellt bzw. gesteuert werden.

Erfindungsgemäß kann die Zusammensetzung nach der Erfindung zudem mindestens einen weiteren Wirk- und/oder Inhaltsstoff, insbesondere pharmazeutischen Zusatz- und/oder Hilfsstoff, enthalten, insbesondere ausgewählt aus der Gruppe von Verarbeitungshilfsstoffen, Stabilisatoren, Emulgatoren, Antioxidantien, Konservierungsstoffen, Feuchthaltemitteln, Süßstoffen und Süßungsmitteln, pH-Stellmitteln, pH-Puffersubstanzen, Verdickungsmitteln, Aromastoffen, Antiseptika, Färbe-, Puffer-, Riech-, Duft-, Streck-, Binde-, Netz- und/oder Konservierungsstoffen sowie deren Kombinationen und Mischungen.

Erfindungsgemäß ist es vorgesehen, dass die Zusammensetzung zumindest im Wesentlichen kein Phenoxyethanol (2-Phenoxyethanol) enthält. Erfindungsgemäß ist es somit vorgesehen, dass die Zusammensetzung zumindest im Wesentlichen frei ist von Phenoxyethanol (2-Phenoxyethanol). Insbesondere kann die Zusammensetzung in Abwesenheit von Phenoxyethanol (2-Phenoxyethanol) vorliegen.

Erfindungsgemäß ist es somit insbesondere vorgesehen, dass die Zusammensetzung kein Phenoxyethanol (2-Phenoxyethanol) aufweist bzw. frei von Phenoxyethanol (2-Phenoxyethanol) ist. Denn die Anmelderin hat in überraschender Weise gefunden, dass die Verwendung von Octenidin bzw. dessen Salze bzw. Ester, insbesondere Octenidindihydrochlorid, mit einer hervorragenden antiviralen Wirkung einhergeht. Hierdurch kann ein weiterführend optimiertes Wirkspektrum bei gleichzeitig geringen Nebenwirkungen bereitgestellt werden.

Auch in diesem Zusammenhang kann es erfindungsgemäß insbesondere vorgesehen sein, dass die Zusammensetzung nach der Erfindung Octenidin und/oder dessen Salze und/oder Ester, insbesondere Octenidindihydrochlorid, als alleinige antivirale Wirksubstanz aufweist bzw. dass die erfindungsgemäße Zusammensetzung frei von von Octenidin und/oder dessen Salzen und/oder Estern, insbesondere Octenidindihydrochlorid, verschiedenen antiviralen Wirkstoffen ist. Erfindungsgemäß kann es insbesondere vorgesehen sein, dass die Zusammensetzung das Octenidin und/oder dessen Salze und/oder Ester, insbesondere Octenidindihydrochlorid als alleinigen Wirkstoff, insbesondere als alleinigen pharmazeutischen Wirkstoff, enthält.

Erfindungsgemäß kann es zusammenfassend insbesondere vorgesehen sein, dass die Zusammensetzung das Octenidin und/oder dessen Salze und/oder Ester, vorzugsweise Octenidindihydrochlorid, als alleinigen antiviralen Wirkstoff, insbesondere als alleinigen pharmazeutischen Wirkstoff, vorzugsweise als alleinigen Wirkstoff, enthält.

Darüber hinaus kann es erfindungsgemäß auch vorgesehen sein, dass die Zusammensetzung im Wesentlichen kein n-Propanol (Propan-1-ol) und/oder zumindest im Wesentlichen kein Isopropanol, insbesondere zumindest im Wesentlichen keinen primären Alkohol, enthält. Erfindungsgemäß kann es dabei insbesondere vorgesehen sein, dass die Zusammensetzung zumindest im Wesentlichen frei ist von n-Propanol (Propan-1-ol) und/oder zumindest im Wesentlichen frei ist von Isopropanol. Insbesondere kann die Zusammensetzung frei sein von einem primären Alkohol. Insbesondere kann die Zusammensetzung in Abwesenheit von n-Propanol (Propan-1-ol) und/oder in Abwesenheit von Isopropanol, insbesondere in Abwesenheit von einem primären Alkohol, vorliegen. Auch hierdurch kann die Verträglichkeit der erfindungsgemäßen Zusammensetzung verbessert werden.

Was die erfindungsgemäße Zusammensetzung weiterhin anbelangt, so kommt auch den absoluten Mengen bzw. der Dosis der jeweils eingesetzten Wirk- bzw. Inhaltsstoffe eine große Bedeutung zu, und zwar auch was die Bereitstellung einer optimierten Wirksamkeit und definierten Anwendungseigenschaften der erfindungsgemäßen Zusammensetzung anbelangt. Der Begriff "Darreichungseinheit" wie dieser im Rahmen der vorliegenden Erfindung insbesondere im Zusammenhang mit den absoluten Mengen bzw. Dosen verwendet wird, bezieht sich dabei insbesondere auf die einem Patienten verabreichte einzelne Applikation (Einzelapplikation) bzw. auf eine einzelne Dosis (Einzeldosis) der hergerichteten Dosierungsform (z. B. Lutschtablette) der Zusammensetzung nach der Erfindung (vgl. auch nachfolgende Ausführungen).

Erfindungsgemäß kann es sich in diesem Zusammenhang insbesondere wie folgt verhalten:
So enthält die Zusammensetzung das Octenidin und/oder dessen Salze und/oder Ester, vorzugsweise Octenidindihydrochlorid, in einer (absoluten) Menge und/oder Dosis im Bereich von 0,05 mg bis 15 mg, insbesondere im Bereich von 0,1 mg bis 12,5 mg, vorzugsweise im Bereich von 0,2 mg bis 10 mg, bevorzugt im Bereich von 0,3 mg bis 7,5 mg, besonders bevorzugt im Bereich von 0,5 mg bis 5 mg, bezogen auf eine Darreichungseinheit der Zusammensetzung.

Zudem kann die Zusammensetzung das Lokalanästhetikum, insbesondere Lidocain und/oder dessen Salze und/oder Ester, vorzugsweise Lidocainhydrochlorid, in einer (absoluten) Menge und/oder Dosis im Bereich von 0,1 mg bis 50 mg, insbesondere im Bereich von 1 mg bis 20 mg, vorzugsweise im Bereich von 3 mg bis 15 mg, bevorzugt im Bereich von 4 mg bis 10 mg, bezogen auf eine Darreichungseinheit der Zusammensetzung, enthalten.

Zudem kann die Zusammensetzung das Antiphlogistikum, insbesondere Benzydamin und/oder dessen Salze und/oder Ester, bevorzugt Benzydaminhydrochlorid, in einer (absoluten) Menge und/oder Dosis im Bereich von 0,1 mg bis 50 mg, insbesondere im Bereich von 0,5 mg bis 20 mg, vorzugsweise im Bereich von 1 mg bis 15 mg, bevorzugt im Bereich von 2 mg bis 10 mg, bezogen auf eine Darreichungseinheit der Zusammensetzung, enthalten.

Darüber hinaus kann die Zusammensetzung das nichtsteroidale Antiphlogistikum (NSAR-Wirkstoff), insbesondere Flurbiprofen und/oder dessen Salze und/oder Ester, bevorzugt Flurbiprofen, in einer (absoluten) Menge und/oder Dosis im Bereich von 0,1 mg bis 50 mg, insbesondere im Bereich von 0,5 mg bis 20 mg, vorzugsweise im Bereich von 1 mg bis 15 mg, bevorzugt im Bereich von 2 mg bis 10 mg, bezogen auf eine Darreichungseinheit der Zusammensetzung, enthalten.

Weiterhin kann die Zusammensetzung das Säuerungsmittel, insbesondere in Form einer organischen Säure und/oder deren Salze und/oder Ester, bevorzugt Weinsäure und/oder Citronensäure, bevorzugt Weinsäure, in einer (absoluten) Menge und/oder Dosis im Bereich von 0,5 mg bis 100 mg, insbesondere im Bereich von 1 mg bis 50 mg, vorzugsweise im Bereich von 2 mg bis 20 mg, bezogen auf eine Darreichungseinheit der Zusammensetzung, enthalten.

Zudem kann die Zusammensetzung das Anisöl, insbesondere Sternanisöl, in einer (absoluten) Menge und/oder Dosis im Bereich von 0,2 mg bis 80 mg, insbesondere im Bereich von 0,5 mg bis 40 mg, vorzugsweise im Bereich von 0,75 mg bis 15 mg, bezogen auf eine Darreichungseinheit der Zusammensetzung, enthalten.

Weiterführend kann die Zusammensetzung das ätherische Öl, insbesondere Pfefferminzöl, in einer (absoluten) Menge und/oder Dosis im Bereich von 0,01 mg bis 5 mg, insbesondere im Bereich von 0,05 mg bis 3 mg, vorzugsweise im Bereich von 0,1 mg bis 1 mg, bezogen auf eine Darreichungseinheit der Zusammensetzung, enthalten.

Darüber hinaus kann die Zusammensetzung den Kaffee-Extrakt und/oder Tee-Extrakt, insbesondere Kaffee-Extrakt, in einer (absoluten) Menge und/oder Dosis im Bereich von 0,05 mg bis 100 mg, insbesondere im Bereich von 0,5 mg bis 50 mg, vorzugsweise im Bereich von 1 mg bis 20 mg, bezogen auf eine Darreichungseinheit der Zusammensetzung, enthalten.

Weiterführend kann die Zusammensetzung das Propylenglykol in einer (absoluten) Menge und/oder Dosis im Bereich von 0,05 mg bis 50 mg, insbesondere im Bereich von 0,5 mg bis 20 mg, vorzugsweise im Bereich von 1 mg bis 10 mg, bezogen auf eine Darreichungseinheit der Zusammensetzung, enthalten.

Nochmals weiterführend kann die Zusammensetzung die Schleimdroge und/oder deren Extrakt in einer (absoluten) Menge und/oder Dosis im Bereich von 0,5 mg bis 200 mg, insbesondere im Bereich von 1 mg bis 100 mg, vorzugsweise im Bereich von 5 mg bis 50 mg, bezogen auf eine Darreichungseinheit der Zusammensetzung, enthalten.

Im Hinblick auf die vorstehenden Angaben zu den (absoluten) Mengen bzw. Dosen verhält es sich insbesondere derart, dass die jeweilige (absolute) Menge und/oder Dosis die mit einer einzelnen Applikation (Einzelapplikation) der Zusammensetzung und/oder die mit einer einzelnen Applikation (Einzelapplikation) einer hergerichteten Dosierungsform der Zusammensetzung verabreichte (Einzel-) Menge und/oder (Einzel-)Dosis ist bzw. dass die Darreichungseinheit eine einzelne Applikation (Einzelapplikation) der Zusammensetzung und/oder eine einzelne Applikation (Einzelapplikation) einer hergerichteten Dosierungsform der Zusammensetzung ist.

Insbesondere kann das Octenidin und/oder dessen Salze und/oder Ester, vorzugsweise Octenidindihydrochlorid, mit einer Tagesdosis im Bereich von 0,15 mg/diem bis 120 mg/diem, insbesondere im Bereich von 0,15 mg/diem bis 120 mg/diem, vorzugsweise im Bereich von 0,3 mg/diem bis 100 mg/diem, bevorzugt im Bereich von 1,5 mg/diem bis 60 mg/diem, besonders bevorzugt im Bereich von 2,25 mg/diem bis 120 mg/diem, weiter bevorzugt im Bereich von 2,5 mg/diem bis 40 mg/diem, verabreicht werden.

Erfindungsgemäß kann die Zusammensetzung somit zur Verabreichung von Octenidin und/oder dessen Salzen und/oder Estern, vorzugsweise Octenidindihydrochlorid, in einer Tagesdosis im Bereich von 0,15 mg/diem bis 120 mg/diem, insbesondere im Bereich von 0,15 mg/diem bis 120 mg/diem, vorzugsweise im Bereich von 0,3 mg/diem bis 100 mg/diem, bevorzugt im Bereich von 1,5 mg/diem bis 60 mg/diem, besonders bevorzugt im Bereich von 2,25 mg/diem bis 120 mg/diem, weiter bevorzugt im Bereich von 2,5 mg/diem bis 40 mg/diem, hergerichtet sein.

Wie auch zuvor angeführt, kommt der Galenik bzw. der Ausbildung der Dosierungsform der erfindungsgemäßen Zusammensetzung gleichermaßen eine große Bedeutung zu:
Im Allgemeinen ist es erfindungsgemäß vorgesehen, dass die Zusammensetzung nach der Erfindung als feste Dosierungsform vorliegt bzw. ausgebildet ist.

Im Rahmen der vorliegenden Erfindung ist es vorgesehen, dass die Zusammensetzung als feste Dosierungsform in Form einer Lutschtablette, vorzugsweise Hartkaramelle, vorliegt und/oder ausgebildet ist bzw. dass die Zusammensetzung als Lutschtablette, vorzugsweise Hartkaramelle, vorliegt bzw. ausgebildet ist.

Die Angaben hinsichtlich des festen Zustands der erfindungsgemäßen Zusammensetzung beziehen sich dabei im Allgemeinen auf die diesbezügliche Ausbildung der Zusammensetzung bei Raumtemperatur (20°C) und Umgebungsdruck (1.013,25 hPa).

Nachfolgend wird die erfindungsgemäß realisierte Ausführungsform weiterführend beschrieben, wonach die Zusammensetzung nach der Erfindung als feste Dosierungsform vorliegt: So ist es erfindungsgemäß vorgesehen, dass die Zusammensetzung als feste Dosierungsform vorliegt bzw. ausgebildet ist.

Dabei liegt die Zusammensetzung nach der Erfindung als feste Dosierungsform in Form einer Lutschtablette, vorzugsweise Hartkaramelle, vor bzw. ist die Zusammensetzung nach der Erfindung als Lutschtablette ausgebildet. Folglich ist es erfindungsgemäß vorgesehen, dass die Zusammensetzung als Lutschtablette, vorzugsweise Hartkaramelle, vorliegt bzw. ausgebildet ist.

Erfindungsgemäß weist die feste Dosierungsform in Form einer Lutschtablette, vorzugsweise Hartkaramelle, ein Gesamtgewicht im Bereich von 0,5 g bis 7 g, insbesondere im Bereich von 1 g bis 6 g, vorzugsweise im Bereich von 2 g bis 5 g, auf. Insbesondere kann die Lutschtablette, vorzugsweise Hartkaramelle, ein Gesamtgewicht im Bereich von 0,5 g bis 7 g, insbesondere im Bereich von 1 g bis 6 g, vorzugsweise im Bereich von 2 g bis 5 g, aufweisen. Die vorgenannten Gewichtsangaben beziehen sich dabei auf eine einzelne Dosierungsform, nämlich eine einzelne Lutschtablette bzw. Hartkaramelle.

Bei der erfindungsgemäßen pharmazeutischen Zusammensetzung in Form einer Lutschtablette, insbesondere auf Hartkaramellenbasis, liegt das Gesamtgewicht der Lutschtablette im Bereich von 0,5 g bis 7 g, insbesondere im Bereich von 1 g bis 6 g, vorzugsweise im Bereich von 2 g bis 5 g.

Erfindungsgemäß ist die Zusammensetzung bzw. die feste Dosierungsform eine Lutschtablette, insbesondere in Form einer Hartkaramelle.

In diesem Zusammenhang ist die Zusammensetzung bzw. die feste Dosierungsform auf Lutschtablettenbasis gebildet bzw. liegt die Zusammensetzung bzw. die feste Dosierungsform als Lutschtablette, insbesondere Hartkaramelle, vor bzw. ist als Lutschtablette, insbesondere Hartkaramelle, ausgebildet. Auf dieser Basis kann die Zusammensetzung bzw. die feste Dosierungsform eine therapeutisch wirksame Menge an Wirk- und/oder Inhaltsstoffen, insbesondere Octenidin und/oder dessen Salze und/oder Ester, vorzugsweise Octenidindihydrochlorid, beim Lutschen freisetzen, wenn die Zusammensetzung bzw. die feste Dosierungsform im Mund- und Rachenraum eines Patienten verabreicht und gelutscht wird.

Im Allgemeinen enthält die erfindungsgemäße pharmazeutische Zusammensetzung bzw. die feste Dosierungsform in Form einer Lutschtablette die Wirk- und/oder Inhaltsstoffe in einer festen Matrix bzw. Masse. Die Wirk- und/oder Inhaltsstoffe sind somit insbesondere in einer Matrix inkorporiert bzw. eingelagert, so dass sie wirksam geschützt und zudem insbesondere homogen verteilt sind.

Die Anmelderin hat gefunden, dass der Wirkstoff Octenidin und/oder dessen Salze bzw. Ester bzw. Octenidindihydrochlorid in Form einer festen galenischen Zubereitung insbesondere zum Lutschen, vorzugsweise in Form einer Lutschtablette, eine optimale Wirksamkeit im Hinblick auf die prophylaktische bzw. therapeutische topische Behandlung der zugrundeliegenden Erkrankungen, insbesondere COVID-19, aufweist. Ohne sich auf diese Theorie beschränken oder berufen zu wollen, geht die erfindungsgemäße Ausführung, wonach die Zusammensetzung als feste Dosierungsform, nämlich in Form einer Lutschtablette, vorliegt, mit einem optimierten Freisetzungsverhalten des Wirkstoffs einher, und zwar sowohl was die zeitbezogene Freisetzungsmenge bzw. die Freisetzungsgeschwindigkeit als auch die Einwirkdauer anbelangt, so dass die antivirale Wirksamkeit auch von daher entsprechend verbessert wird.

Die feste Dosierungsform zum Lutschen und die Inkorporierung der Wirk- und/oder Inhaltsstoffe in eine feste Matrix bzw. Masse bietet in diesem Zusammenhang eine Reihe von Vorteilen: Zum einen lassen sich auf diese Weise die Wirk- und Inhaltsstoffe besser dosieren, d. h. es wird eine verbesserte Dosiergenauigkeit erreicht. Zum anderen wird dadurch die Applikations- bzw. Einnahmemöglichkeit verbessert, d. h. die Einnahmemöglichkeit für den Patienten ist sozusagen überall gegeben (z. B. unterwegs auf Reisen, im Freien etc.), da keine speziellen Zubereitungsformen angemischt werden müssen. Darüber hinaus liefert die feste Dosierungsform den Vorteil, dass die Wirkstoffe in festen Zubereitungen im allgemeinen lagerstabil sind. Weiterhin gewährt die feste Dosierungsform eine definierte Verweilzeit bzw. Verweildauer im Mund- und Rachenraum und somit eine effiziente und kontrollierte Therapierung. Schließlich ermöglicht die feste Dosierungsform, insbesondere durch die Einlagerung der Wirk- und Inhaltsstoffe in die Matrix, eine verbesserte Kombination mit anderen Wirkstoffen und deren gleichmäßige, homogene Verteilung über die Matrix.

Erfindungsgemäß enthält die Zusammensetzung bzw. die feste Dosierungsform mindestens einen Zucker und/oder mindestens einen Zuckeraustauschstoff, und zwar als Exzipient (Träger), vorzugsweise pharmakologisch und/oder physiologisch unbedenklicher Exzipient (Träger).

Als Matrix bzw. Masse für die Einlagerung der Wirk- und Inhaltsstoffe eignen sich insbesondere Zucker aller Art und/oder Zuckeraustauschstoffe aller Art.

In diesem Zusammenhang kann der Zucker ausgewählt sein aus der Gruppe von Saccharose, Glucose, insbesondere Dextrose, und Fructose.

Zudem kann der Zuckeraustauschstoff ausgewählt sein aus der Gruppe von Zuckeralkoholen, vorzugsweise aus der Gruppe von Mannit, Xylit, Sorbit, Isomalt, Maltitsirup, Lactit, Leucrose, Fructooligosacchariden, Glucanen, Polyglucose sowie deren Kombinationen und Mischungen, besonders bevorzugt Isomalt und Mannit sowie deren Kombinationen und Mischungen, ganz besonders bevorzugt Isomalt. Auf dieser Basis können kalorienreduzierte bzw. zahnfreundliche bzw. nichtkariogene Zusammensetzungen bereitgestellt werden.

Erfindungsgemäß ist es im Hinblick auf die feste Dosierungsform somit insbesondere vorgesehen, dass die Zusammensetzung, insbesondere die feste Dosierungsform, die Wirk- und/oder Inhaltsstoffe in einer festen Matrix und/oder Masse, insbesondere in einer Matrix und/oder Masse auf Basis von Zuckern und/oder Zuckeraustauschstoffen, insbesondere wie zuvor definiert, aufweist.

Insbesondere kann die Zusammensetzung, insbesondere die feste Dosierungsform, den Zucker und/oder den Zuckeraustauschstoff in einer (relativen) Menge im Bereich von 50 Gew.-% bis 99,95 Gew.-%, insbesondere im Bereich von 70 Gew.-% bis 99,5 Gew.-%, vorzugsweise im Bereich von 80 Gew.-% bis 99,25 Gew.-%, bevorzugt im Bereich von 90 Gew.-% bis 99 Gew.-%, bezogen auf die Zusammensetzung, insbesondere die feste Dosierungsform, aufweisen.

Insbesondere bilden die Zucker und/oder Zuckeralkohole die Matrix und/oder die feste Masse der als feste Dosierungsform vorliegenden Zusammensetzung aus. Somit kann die Menge an Matrixmasse (d. h. Zuckern und/oder Zuckeraustauschstoffen) im Allgemeinen im Bereich von 50 Gew.-% bis 99,95 Gew.-%, insbesondere im Bereich von 70 Gew.-% bis 99,5 Gew.-%, vorzugsweise im Bereich von 80 Gew.-% bis 99,25 Gew.-%, bevorzugt im Bereich von 90 Gew.-% bis 99 Gew.-%, bezogen auf die Zusammensetzung, insbesondere die feste Dosierungsform, liegen.

Während Zucker und Süßstoffe gemeinsam als Süßungsmittel bezeichnet werden, werden - im Gegensatz zu den intensiv schmeckenden Süßstoffen - Zuckeraustauschstoffe technologisch wie Saccharose eingesetzt, d. h. sie besitzen einen "Körper" und einen physiologischen Brennwert ("nutritive Zuckeraustauschstoffe"). Die Süßkraft entspricht in weiten Grenzen etwa der von Saccharose. Der physiologische Vorteil der Zuckeraustauschstoffe im Vergleich zu Saccharose liegt in der insulinunhabhängigen Metabolisierung (vorteilhaft z. B. für Diabetiker) und in der zum Teil verminderten kariogenen Wirkung. Für einige Zuckeraustauschstoffe (z. B. Xylit) ist eine antikariogene Wirkung beschrieben.

Der Begriff "Zuckeralkohol" ist die Gruppenbezeichnung für die aus Monosacchariden durch Reduktion der Carbonylgruppe entstehenden Polyhydroxyverbindungen, die keine Zucker sind, gleichwohl aber süß schmecken und deshalb gegebenenfalls Verwendung als Zuckeraustauschstoffe finden können. Man unterscheidet bei diesen im allgemeinen kristallinen, wasserlöslichen Polyolen nach der Anzahl der im Molekül enthaltenen Hydroxygruppen sogenannte Tetrite, Pentite, Hexite usw. Natürlich vorkommende Zuckeralkohole sind z. B. Glycerin, Threit und Erythrit, Adonit (Ribit), Arabit (früher: Lyxit) und Xylit, Dulcit (Galactit), Mannit und Sorbit (Glucit).

Für weitergehende Einzelheiten zu den Begriffen "Zucker", "Zuckeraustauschstoffe" und "Zuckeralkohole" kann beispielsweise verwiesen werden auf Römpp Chemie-Lexikon, 10. Auflage, Band 6, Georg Thieme Verlag, Stuttgart/New York, 1999, Seiten 5096 bis 5100, Stichworte: "Zucker", "Zuckeralkohole" und "Zuckeraustauschstoffe".

Weiterhin kommt auch der Restfeuchte der Zusammensetzung in Form der festen Dosierungsform eine große Bedeutung zu. Insbesondere kann die Zusammensetzung bzw. die feste Dosierungsform eine Restfeuchte bzw. einen Restfeuchtegehalt von höchstens 10 Gew.-%, insbesondere höchstens 5 Gew.-%, vorzugsweise höchstens 3 Gew.-%, bezogen auf die Zusammensetzung in Form der festen Dosierungsform, aufweisen. Gleichermaßen kann die Zusammensetzung bzw. die feste Dosierungsform eine Restfeuchte bzw. einen Restfeuchtegehalt im Bereich von 0,1 Gew.-% bis 10 Gew.-%, insbesondere im Bereich von 0,5 Gew.-% bis 5 Gew.-%, vorzugsweise im Bereich von 1 Gew.-% bis 3 Gew.-%, bezogen auf die Zusammensetzung in Form der festen Dosierungsform, aufweisen.

Im Zusammenhang mit dem definierten Restfeuchtegehalt liegen auch optimale Zerfalls- bzw. Freisetzungseigenschaften bei oraler Applikation der Zusammensetzung nach der Erfindung in Form der festen Dosierungsform vor. Zudem ist die (Lagerungs-)Stabilität erhöht.

Die Herstellung der erfindungsgemäßen pharmazeutischen Zusammensetzung erfolgt in an sich bekannter Weise. Für weitergehende diesbezügliche Einzelheiten kann auch auf die nachfolgenden Ausführungsbeispiele verwiesen werden.

Bei der Herstellung von Lutschtabletten auf Hartkaramellenbasis kann beispielsweise derart vorgegangen werden, dass zunächst die Wirk- und Inhaltsstoffe - gegebenenfalls nach Zerkleinerung - eingewogen werden und dann in die zuvor erwärmte Grundsubstanz auf Basis von Zuckern oder Zuckerersatzstoffen (z. B. Isomalt) eingemischt werden, gefolgt von einer Formung von Lutschtabletten und nachfolgendem Abkühlen. Derartige Herstellverfahren sind dem Fachmann als solche wohlbekannt.

Die Herstellung von Hartkaramellen kann typischerweise wie folgt durchgeführt werden: Bei der Herstellung von erfindungsgemäßen Lutschtabletten, insbesondere in Form von Hartkaramellen, werden zunächst der Zucker bzw. die Zuckerersatzstoffe bzw. Zuckeraustauschstoffe in Wasser gelöst, anschließend bei Temperaturen erwärmt (z. B. auf Temperaturen zwischen 120 und 140 °C) und schließlich vakuumiert. Dieser erwärmten bzw. heißen Masse können dann die Wirk- und Inhaltsstoffe, d. h. Octenidin sowie gegebenenfalls weitere Inhaltsstoffe, wie z. B. Lokalanästhetikum, Schleimdrogen bzw. deren Extrakte, Farbstoffe, Aromen, Süßstoffe etc., in fester oder flüssiger Form zudosiert werden. Nach kontrolliertem Abkühlen (z. B. auf Temperaturen unterhalb von etwa 75 °C) können die Hartkaramellen in der gewünschten Form geprägt werden. Die Formen können - wie gewünscht - beispielsweise rund oder polygonal sein. Abschließend können die Hartkaramellen kontrolliert auf Raumtemperatur abgekühlt und sortiert werden. Die Verpackung der auf diese Weise hergestellten Hartkaramellen kann vereinzelt in Blistern oder Sachets oder auch gesammelt in Beuteln bzw. Tüten erfolgen.

Gemäß dem vorliegenden Aspekt betrifft die vorliegende Erfindung auch die nachfolgenden Zusammensetzungen, welche gleichermaßen in Form einer festen Dosierungsform vorliegen:
So betrifft die vorliegende Erfindung gemäß dem vorliegenden Aspekt auch eine erfindungsgemäße Zusammensetzung, insbesondere pharmazeutische Zusammensetzung, zur Verwendung bei der prophylaktischen und/oder therapeutischen topischen (lokalen) Behandlung, insbesondere prophylaktischen und/oder therapeutischen antiviralen topischen Behandlung, von durch Corona-Viren, vorzugsweise SARS-CoV-2, hervorgerufenen Viruserkrankungen, bevorzugt von COVID-19, und zur topischen Anwendung auf die Schleimhäute des Mund-, Rachen- und/oder Nasenraums, insbesondere eine wie zuvor definierte Zusammensetzung zur Verwendung,
wobei die Zusammensetzung als antiviralen Wirkstoff Octenidin und/oder dessen Salze und/oder Ester, vorzugsweise Octenidindihydrochlorid, enthält,
wobei die pharmazeutische Zusammensetzung als feste Dosierungsform in Form einer Lutschtablette, insbesondere auf Hartkaramellenbasis und/oder als Hartkaramelle, vorliegt und/oder ausgebildet ist, wobei die Lutschtablette ein Gesamtgewicht im Bereich von 0,5 g bis 7 g, insbesondere im Bereich von 1 g bis 6 g, vorzugsweise im Bereich von 2 g bis 5 g, aufweist, enthaltend je Lutschtablette:
   - Octenidin und/oder dessen Salze und/oder Ester, vorzugsweise Octenidindihydrochlorid, in einer (absoluten) Menge und/oder Dosis im Bereich von 0,05 mg bis 15 mg, insbesondere im Bereich von 0,1 mg bis 12,5 mg, vorzugsweise im Bereich von 0,5 mg bis 10 mg, bevorzugt im Bereich von 0,75 mg bis 7,5 mg, besonders bevorzugt im Bereich von 1 mg bis 5 mg;
   - gegebenenfalls mindestens einen weiteren Wirk- und/oder Inhaltsstoff, insbesondere pharmazeutischen Zusatz- und/oder Hilfsstoff, insbesondere ausgewählt aus der Gruppe von Verarbeitungshilfsstoffen, Stabilisatoren, Emulgatoren, Antioxidantien, Konservierungsstoffen, Feuchthaltemitteln, Süßstoffen und Süßungsmitteln, pH-Stellmitteln, pH-Puffersubstanzen, Verdickungsmitteln, Aromastoffen, Antiseptika, Färbe-, Puffer-, Riech-, Duft-, Streck-, Binde-, Netz- und/oder Konservierungsstoffen sowie deren Kombinationen und Mischungen, in Mengen im Bereich von 0,1 mg bis 500 mg, insbesondere im Bereich von 0,5 mg bis 250 mg, vorzugsweise im Bereich von 1 mg bis 100 mg;
   - mindestens einen Zucker und/oder mindestens einen Zuckeraustauschstoff als Exzipient (Träger), vorzugsweise pharmakologisch und/oder physiologisch unbedenklicher Exzipient (Träger); wobei der Zucker ausgewählt ist aus der Gruppe von Saccharose, Glucose, insbesondere Dextrose, und Fructose und/oder wobei der Zuckeraustauschstoff ausgewählt ist aus Zuckeralkoholen, vorzugsweise aus der Gruppe von Mannit, Xylit, Sorbit, Isomalt, Maltitsirup, Lactit, Leucrose, Fructooligosacchariden, Glucanen, Polyglucose und deren Kombinationen und Mischungen, besonders bevorzugt Isomalt und/oder Mannit, ganz besonders bevorzugt Isomalt; und in Mengen zur Bereitstellung und/oder Erhalt des Gesamtgewichts der Lutschtablette,
wobei die Lutschtablette zumindest im Wesentlichen frei ist von Phenoxyethanol.

Weiterhin betrifft die vorliegende Erfindung gemäß dem vorliegenden Aspekt auch eine erfindungsgemäße Zusammensetzung, insbesondere pharmazeutische Zusammensetzung, zur Verwendung bei der prophylaktischen und/oder therapeutischen topischen (lokalen) Behandlung, insbesondere prophylaktischen und/oder therapeutischen antiviralen topischen Behandlung, von durch Corona-Viren, vorzugsweise SARS-CoV-2, hervorgerufenen Viruserkrankungen, bevorzugt von COVID-19, und zur topischen Anwendung auf die Schleimhäute des Mund-, Rachen- und/oder Nasenraums, insbesondere eine wie zuvor definierte Zusammensetzung zur Verwendung,
wobei die Zusammensetzung als feste Dosierungsform in Form einer Lutschtablette, insbesondere auf Hartkaramellenbasis und/oder als Hartkaramelle, vorliegt und/oder ausgebildet ist, wobei die Lutschtablette ein Gesamtgewicht im Bereich von 0,5 bis 7 g, insbesondere im Bereich von 1 bis 6 g, vorzugsweise im Bereich von 2 bis 5 g, aufweist, enthaltend je Lutschtablette:
   - Octenidin und/oder dessen Salze und/oder Ester, vorzugsweise Octenidindihydrochlorid, in einer (absoluten) Menge und/oder Dosis im Bereich von 0,05 mg bis 15 mg, insbesondere im Bereich von 0,1 mg bis 12,5 mg, vorzugsweise im Bereich von 0,5 mg bis 10 mg, bevorzugt im Bereich von 0,75 mg bis 7,5 mg, besonders bevorzugt im Bereich von 1 mg bis 5 mg;
   - gegebenenfalls mindestens ein Lokalanästhetikum, insbesondere Lidocain, bevorzugt Lidocainhydrochlorid, in einer (absoluten) Menge und/oder Dosis im Bereich von 0,1 mg bis 50 mg, insbesondere im Bereich von 1 mg bis 20 mg, vorzugsweise im Bereich von 3 mg bis 15 mg, bevorzugt im Bereich von 4 mg bis 10 mg;
   - gegebenenfalls mindestens einen weiteren Wirk- und/oder Inhaltsstoff, insbesondere pharmazeutischen Zusatz- und/oder Hilfsstoff, insbesondere ausgewählt aus der Gruppe von Verarbeitungshilfsstoffen, Stabilisatoren, Emulgatoren, Antioxidantien, Konservierungsstoffen, Feuchthaltemitteln, Süßstoffen und Süßungsmitteln, pH-Stellmitteln, pH-Puffersubstanzen, Verdickungsmitteln, Aromastoffen, Antiseptika, Färbe-, Puffer-, Riech-, Duft-, Streck-, Binde-, Netz- und/oder Konservierungsstoffen sowie deren Kombinationen und Mischungen, in Mengen im Bereich von 0,1 mg bis 500 mg, insbesondere im Bereich von 0,5 mg bis 250 mg, vorzugsweise im Bereich von 1 mg bis 100 mg;
   - mindestens einen Zucker und/oder mindestens einen Zuckeraustauschstoff als Exzipient (Träger), vorzugsweise pharmakologisch und/oder physiologisch unbedenklicher Exzipient (Träger); wobei der Zucker ausgewählt ist aus der Gruppe von Saccharose, Glucose, insbesondere Dextrose, und Fructose; und/oder wobei der Zuckeraustauschstoff ausgewählt ist aus Zuckeralkoholen, vorzugsweise aus der Gruppe von Mannit, Xylit, Sorbit, Isomalt, Maltitsirup, Lactit, Leucrose, Fructooligosacchariden, Glucanen, Polyglucose sowie deren Kombinationen und Mischungen, besonders bevorzugt Isomalt und/oder Mannit, ganz besonders bevorzugt Isomalt; und in Mengen zur Bereitstellung und/oder Erhalt des Gesamtgewichts der Lutschtablette,
wobei die Lutschtablette zumindest im Wesentlichen frei ist von Phenoxyethanol (2-Phenoxyethanol).

Gleichermaßen betrifft die vorliegende Erfindung gemäß dem vorliegenden Aspekt auch eine erfindungsgemäße Zusammensetzung, insbesondere pharmazeutische Zusammensetzung, zur Verwendung bei der prophylaktischen und/oder therapeutischen topischen (lokalen) Behandlung, insbesondere prophylaktischen und/oder therapeutischen antiviralen topischen Behandlung, von durch Corona-Viren, vorzugsweise SARS-CoV-2, hervorgerufenen Viruserkrankungen, bevorzugt von COVID-19, und zur topischen Anwendung auf die Schleimhäute des Mund-, Rachen- und/oder Nasenraums, insbesondere eine wie zuvor definierte Zusammensetzung zur Verwendung,
wobei die Zusammensetzung als feste Dosierungsform in Form einer Lutschtablette, insbesondere auf Hartkaramellenbasis und/oder als Hartkaramelle, vorliegt und/oder ausgebildet ist, wobei die Lutschtablette ein Gesamtgewicht im Bereich von 0,5 g bis 7 g, insbesondere im Bereich von 1 g bis 6 g, vorzugsweise im Bereich von 2 g bis 5 g, aufweist, enthaltend je Lutschtablette:
   - Octenidin und/oder dessen Salze und/oder Ester, vorzugsweise Octenidindihydrochlorid, in einer (absoluten) Menge und/oder Dosis im Bereich von 0,05 mg bis 15 mg, insbesondere im Bereich von 0,1 mg bis 12,5 mg, vorzugsweise im Bereich von 0,5 mg bis 10 mg, bevorzugt im Bereich von 0,75 mg bis 7,5 mg, besonders bevorzugt im Bereich von 1 mg bis 5 mg;
   - gegebenenfalls mindestens ein Lokalanästhetikum, insbesondere Lidocain, bevorzugt Lidocainhydrochlorid, in einer (absoluten) Menge und/oder Dosis im Bereich von 0,1 mg bis 50 mg, insbesondere im Bereich von 1 mg bis 20 mg, vorzugsweise im Bereich von 3 mg bis 15 mg, bevorzugt im Bereich von 4 mg bis 10 mg;
   - gegebenenfalls mindestens ein Antiphlogistikum, insbesondere Benzydamin und/oder dessen Salze und/oder Ester, bevorzugt Benzydaminhydrochlorid, in einer (absoluten) Menge und/oder Dosis im Bereich von 0,1 mg bis 50 mg, insbesondere im Bereich von 0,5 mg bis 20 mg, vorzugsweise im Bereich von 1 mg bis 15 mg, bevorzugt im Bereich von 2 mg bis 10 mg,
   - gegebenenfalls mindestens ein nichtsteroidales Antiphlogistikum (NSAR-Wirkstoff), insbesondere Flurbiprofen und/oder dessen Salze und/oder Ester, bevorzugt Flurbiprofen, in einer (absoluten) Menge und/oder Dosis im Bereich von 0,1 mg bis 50 mg, insbesondere im Bereich von 0,5 mg bis 20 mg, vorzugsweise im Bereich von 1 mg bis 15 mg, bevorzugt im Bereich von 2 mg bis 10 mg;
   - gegebenenfalls mindestens ein Säuerungsmittel, insbesondere in Form einer organischen Säure oder deren Salze oder Ester, bevorzugt Weinsäure und/oder Citronensäure, bevorzugt Weinsäure, in einer (absoluten) Menge und/oder Dosis im Bereich von 0,5 mg bis 100 mg, insbesondere im Bereich von 1 mg bis 50 mg, vorzugsweise im Bereich von 2 mg bis 20 mg;
   - gegebenenfalls mindestens ein Anisöl, insbesondere Sternanisöl, in einer (absoluten) Menge und/oder Dosis im Bereich von 0,2 mg bis 80 mg, insbesondere im Bereich von 0,5 mg bis 40 mg, vorzugsweise im Bereich von 0,75 mg bis 15 mg;
   - gegebenenfalls mindestens ein ätherisches Öl, insbesondere Pfefferminzöl, in einer (absoluten) Menge und/oder Dosis im Bereich von 0,01 mg bis 5 mg, insbesondere im Bereich von 0,05 mg bis 3 mg, vorzugsweise im Bereich von 0,1 mg bis 1 mg;
   - gegebenenfalls mindestens ein Kaffee-Extrakt und/oder Tee-Extrakt, insbesondere Kaffee-Extrakt, in einer (absoluten) Menge und/oder Dosis im Bereich von 0,05 mg bis 100 mg, insbesondere im Bereich von 0,5 mg bis 50 mg, vorzugsweise im Bereich von 1 mg bis 20 mg;
   - gegebenenfalls Propylenglykol in einer (absoluten) Menge und/oder Dosis im Bereich von 0,05 mg bis 50 mg, insbesondere im Bereich von 0,5 mg bis 20 mg, vorzugsweise im Bereich von 1 mg bis 10 mg;
   - gegebenenfalls mindestens eine Schleimdroge und/oder deren Extrakt in einer (absoluten) Menge und/oder Dosis im Bereich von 0,5 mg bis 200 mg, insbesondere im Bereich von 1 mg bis 100 mg, vorzugsweise im Bereich von 5 mg bis 50 mg;
   - gegebenenfalls mindestens einen weiteren Wirk- und/oder Inhaltsstoff, insbesondere pharmazeutischen Zusatz- und/oder Hilfsstoff, insbesondere ausgewählt aus der Gruppe von Verarbeitungshilfsstoffen, Stabilisatoren, Emulgatoren, Antioxidantien, Konservierungsstoffen, Feuchthaltemitteln, Süßstoffen und Süßungsmitteln, pH-Stellmitteln, pH-Puffersubstanzen, Verdickungsmitteln, Aromastoffen, Antiseptika, Färbe-, Puffer-, Riech-, Duft-, Streck-, Binde-, Netz- und/oder Konservierungsstoffen sowie deren Kombinationen und Mischungen, in einer (absoluten) Menge und/oder Dosis im Bereich von 0,1 mg bis 500 mg, insbesondere im Bereich von 0,5 mg bis 250 mg, vorzugsweise im Bereich von 1 mg bis 100 mg;
   - mindestens einen Zucker und/oder mindestens einen Zuckeraustauschstoff als Exzipient (Träger), vorzugsweise pharmakologisch und/oder physiologisch unbedenklicher Exzipient (Träger); wobei der Zucker ausgewählt ist aus der Gruppe von Saccharose, Glucose, insbesondere Dextrose, und Fructose und/oder wobei der Zuckeraustauschstoff ausgewählt ist aus Zuckeralkoholen, vorzugsweise aus der Gruppe von Mannit, Xylit, Sorbit, Isomalt, Maltitsirup, Lactit, Leucrose, Fructooligosacchariden, Glucanen, Polyglucose sowie deren Kombinationen und Mischungen, besonders bevorzugt Isomalt und/oder Mannit sowie deren Kombinationen und Mischungen, ganz besonders bevorzugt Isomalt; und in Mengen zur Bereitstellung und/oder Erhalt des Gesamtgewichts der Lutschtablette,
wobei die Lutschtablette zumindest im Wesentlichen frei ist von Phenoxyethanol (2-Phenoxyethanol).

Darüber hinaus kann es sich in Bezug auf die erfindungsgemäße Zusammensetzung im Allgemeinen im Rahmen der vorliegenden Erfindung wie folgt verhalten.

Insbesondere kann die Zusammensetzung, wie zuvor definiert, und/oder das Octenidin und/oder dessen Salze und/oder Ester, vorzugsweise Octenidindihydrochlorid, als Co-Therapeutikum und/oder als Co-Medikation im Rahmen einer COVID-19-Basistherapie eingesetzt wird und/oder wobei die Zusammensetzung und/oder das Octenidin und/oder dessen Salze und/oder Ester, vorzugsweise Octenidindihydrochlorid, als Kombinationstherapeutikum zu einer COVID-19-Basistherapie und/oder bestehenden COVID-19-Therapie eingesetzt werden.

Erfindungsgemäß kann die Zusammensetzung und/oder das Octenidin und/oder dessen Salze und/oder Ester, vorzugsweise Octenidindihydrochlorid, zur Expositionsprophylaxe, insbesondere Postexpositionsprophylaxe und/oder zur Präexpositionsprophylaxe, gegenüber Corona-Viren, vorzugsweise SARS-CoV-2, eingesetzt werden.

Weiterhin kann die Zusammensetzung und/oder das Octenidin und/oder dessen Salze und/oder Ester, vorzugsweise Octenidindihydrochlorid, zur Infektionsprophylaxe gegenüber Corona-Viren, vorzugsweise SARS-CoV-2, eingesetzt werden.

Erfindungsgemäß kann es weiterhin vorgesehen sein, dass die Zusammensetzung und/oder das Octenidin und/oder dessen Salze und/oder Ester, vorzugsweise Octenidindihydrochlorid, zur Verringerung der Virenlast von Corona-Viren, vorzugsweise SARS-CoV-2, bevorzugt zur Verringerung der Virenlast im Mund-, Rachen- und/oder Nasenraum, eingesetzt wird.

Im Allgemeinen kann die Zusammensetzung und/oder das Octenidin und/oder dessen Salze und/oder Ester, vorzugsweise Octenidindihydrochlorid, im präsymptomatischen Stadium und/oder postsymptomatischen Stadium, insbesondere im präsymptomatischen Stadium, von insbesondere durch Corona-Viren, vorzugsweise SARS-CoV-2, hervorgerufenen Viruserkrankungen, bevorzugt von COVID-19, eingesetzt werden.

Weiterhin kann die Zusammensetzung und/oder das Octenidin und/oder dessen Salze und/oder Ester, vorzugsweise Octenidindihydrochlorid, bei asymptomatischen Verläufen von durch Corona-Viren, vorzugsweise SARS-CoV-2, hervorgerufenen Viruserkrankungen, bevorzugt von COVID-19, eingesetzt werden.

Darüber hinaus kann die Zusammensetzung und/oder das Octenidin und/oder dessen Salze und/oder Ester, vorzugsweise Octenidindihydrochlorid, bei symptomatischen Verläufen von durch Corona-Viren, vorzugsweise SARS-CoV-2, hervorgerufenen Viruserkrankungen, bevorzugt von COVID-19, eingesetzt werden.

Erfindungsgemäß kann es sich darüber hinaus insbesondere derart verhalten, dass die Zusammensetzung, insbesondere das Octenidin und/oder dessen Salze und/oder Ester, vorzugsweise Octenidindihydrochlorid, die Corona-Viren, vorzugsweise SARS-CoV-2, inaktiviert bzw. abtötet.

Darüber hinaus kann es sich erfindungsgemäß auch derart verhalten, dass die Zusammensetzung, insbesondere das Octenidin und/oder dessen Salze und/oder Ester, vorzugsweise Octenidindihydrochlorid, die Vermehrung und/oder Replikation der Corona-Viren, vorzugsweise von SARS-CoV-2, hemmt und/oder vermindert bzw. unterbindet.

Die vorliegende Erfindung sowohl gemäß dem ersten Aspekt der vorliegenden Erfindung als auch gemäß sämtlichen übrigen vorliegend beschriebenen Aspekten ist somit mit einer Vielzahl von Vorteilen und Besonderheiten verbunden, welche das erfindungsgemäße Therapiekonzept einzigartig und besonders, insbesondere hocheffizient, machen.

Vorliegend beschrieben - gemäß einem vorliegend beschriebenen Aspekt - ist die Verwendung einer Zusammensetzung, insbesondere pharmazeutischen Zusammensetzung, zur Herstellung eines Arzneimittels oder Medikaments zur prophylaktischen und/oder therapeutischen topischen (lokalen) Behandlung, insbesondere prophylaktischen und/oder therapeutischen antiviralen topischen Behandlung, von viralen Erkrankungen, insbesondere von durch Corona-Viren, vorzugsweise SARS-CoV-2, hervorgerufenen (Virus-)Erkrankungen, bevorzugt von COVID-19, insbesondere zur topischen Anwendung im Mund-, Rachen- und/oder Nasenraum und/oder zur topischen Anwendung auf die Schleimhäute des Mund-, Rachen- und/oder Nasenraums, wobei die Zusammensetzung als antiviralen Wirkstoff Octenidin und/oder dessen Salze und/oder Ester, vorzugsweise Octenidindihydrochlorid, enthält.

Gemäß dem vorliegenden Aspekt ist auch beschrieben die Verwendung einer Zusammensetzung, insbesondere pharmazeutischen Zusammensetzung, zur prophylaktischen und/oder therapeutischen topischen (lokalen) Behandlung, insbesondere prophylaktischen und/oder therapeutischen antiviralen topischen Behandlung, von viralen Erkrankungen, insbesondere von durch Corona-Viren, vorzugsweise SARS-CoV-2, hervorgerufenen (Virus-) Erkrankungen, bevorzugt von COVID-19, insbesondere zur topischen Anwendung im Mund-, Rachen- und/oder Nasenraum und/oder zur topischen Anwendung auf die Schleimhäute des Mund-, Rachen- und/oder Nasenraums, wobei die Zusammensetzung als antiviralen Wirkstoff Octenidin und/oder dessen Salze und/oder Ester, vorzugsweise Octenidindihydrochlorid, enthält.

Für weitergehende Einzelheiten zu diesem Aspekt kann Bezug genommen werden auf die Ausführungen zu den weiteren (Erfindungs-)Aspekten, wobei diese Ausführungen gleichermaßen für den vorliegenden Aspekt gelten.

Gleichermaßen ist vorliegend beschrieben - gemäß einem vorliegend beschriebenen Aspekt - Octenidin und/oder dessen Salze und/oder Ester, vorzugsweise Octenidindihydrochlorid, zur Verwendung bei der prophylaktischen und/oder therapeutischen topischen (lokalen) Behandlung, insbesondere prophylaktischen und/oder therapeutischen antiviralen topischen Behandlung, von viralen Erkrankungen, insbesondere von durch Corona-Viren, vorzugsweise SARS-CoV-2, hervorgerufenen (Virus-)Erkrankungen, bevorzugt von COVID-19, insbesondere zur topischen Anwendung im Mund-, Rachen- und/oder Nasenraum und/oder zur topischen Anwendung auf die Schleimhäute des Mund-, Rachen- und/oder Nasenraums.

Für weitergehende Einzelheiten zu diesem Aspekt kann Bezug genommen werden auf die Ausführungen zu den weiteren (Erfindungs-)Aspekten, wobei diese Ausführungen gleichermaßen für den vorliegenden Aspekt gelten.

Wiederum vorliegend beschrieben - gemäß einem vorliegend beschriebenen Aspekt - ist die erfindungsgemäße Verwendung von Octenidin und/oder dessen Salze und/oder Ester, vorzugsweise Octenidindihydrochlorid, zur Herstellung eines Arzneimittels und/oder Medikaments zur prophylaktischen und/oder therapeutischen topischen (lokalen) Behandlung, insbesondere prophylaktischen und/oder therapeutischen antiviralen topischen Behandlung, von viralen Erkrankungen, insbesondere von durch Corona-Viren, vorzugsweise SARS-CoV-2, hervorgerufenen (Virus-)Erkrankungen, bevorzugt von COVID-19, insbesondere zur topischen Anwendung im Mund-, Rachen- und/oder Nasenraum und/oder zur topischen Anwendung auf die Schleimhäute des Mund-, Rachen- und/oder Nasenraums.

In diesem Zusammenhang betrifft die vorliegenden Erfindung gemäß dem vorliegenden Aspekt auch die Verwendung von Octenidin und/oder dessen Salze und/oder Ester, vorzugsweise Octenidindihydrochlorid, zur prophylaktischen und/oder therapeutischen topischen (lokalen) Behandlung, insbesondere prophylaktischen und/oder therapeutischen antiviralen topischen Behandlung, von viralen Erkrankungen, insbesondere von durch Corona-Viren, vorzugsweise SARS-CoV-2, hervorgerufenen (Virus-)Erkrankungen, bevorzugt von COVID-19, insbesondere zur topischen Anwendung im Mund-, Rachen- und/oder Nasenraum und/oder zur topischen Anwendung auf die Schleimhäute des Mund-, Rachen- und/oder Nasenraums.

Für weitergehende Einzelheiten zu diesem Aspekt kann Bezug genommen werden auf die Ausführungen zu den weiteren (Erfindungs-)Aspekten, wobei diese Ausführungen gleichermaßen für den vorliegenden Aspekt gelten.

Was die beiden unmittelbar vorstehend genannten Aspekte zudem anbelangt, so kann das Octenidin und/oder dessen Salze und/oder Ester, vorzugsweise Octenidindihydrochlorid, in einer Zusammensetzung, wie zuvor definiert, eingesetzt bzw. verabreicht werden.

Ebenfalls vorliegend beschrieben - gemäß einem vorliegend beschriebenen Aspekt der vorliegenden Erfindung - ist zudem das erfindungsgemäße Verfahren zur prophylaktischen und/oder therapeutischen topischen (lokalen) Behandlung, insbesondere prophylaktischen und/oder therapeutischen antiviralen topischen Behandlung, von viralen Erkrankungen, insbesondere von durch Corona-Viren, vorzugsweise SARS-CoV-2, hervorgerufenen (Virus-)Erkrankungen, bevorzugt von COVID-19,
wobei bei dem Verfahren einem an einer viralen Erkrankung, insbesondere an einer durch Corona-Viren, vorzugsweise SARS-CoV-2, hervorgerufenen (Virus-) Erkrankung, bevorzugt von COVID-19, erkrankten Patienten eine pharmazeutisch und/oder therapeutisch wirksame Menge, insbesondere pharmazeutisch und/oder therapeutisch antiviral wirksame Menge, an Octenidin und/oder dessen Salze und/oder Ester, vorzugsweise Octenidindihydrochlorid, verabreicht wird.

Für weitergehende Einzelheiten zu diesem Aspekt kann Bezug genommen werden auf die Ausführungen zu den weiteren (Erfindungs-)Aspekten, wobei diese Ausführungen gleichermaßen für den vorliegenden Aspekt gelten.

Was die vorgenannten Aspekte gemäß den drei unmittelbar vorgenannten Aspekten anbelangt, so kann diesbezüglich auch Folgendes vorgesehen sein:
So kann es insbesondere vorgesehen sein, dass das Octenidin und/oder dessen Salze und/oder Ester in Form eines Hydrochloridsalzes, vorzugsweise Octenidindihydrochlorid, eingesetzt bzw. verwendet wird. Zudem kann das Octenidin und/oder dessen Salze und/oder Ester, vorzugsweise Octenidindihydrochlorid, in pharmazeutisch und/oder therapeutisch wirksamen, insbesondere in pharmazeutisch und/oder therapeutisch antiviral wirksamen Mengen, vorliegt und/oder verwendet werden.

Im Hinblick auf die drei unmittelbar vorgenannten Aspekte kann es gleichermaßen vorgesehen sein, dass das Octenidin und/oder dessen Salze und/oder Ester, vorzugsweise Octenidindihydrochlorid, gemeinsam mit mindestens einem Lokalanästhetikum eingesetzt und/oder verwendet wird. Dabei kann das Lokalanästhetikum ein Lokalanästhetikum auf Basis eines organischen Säureesters oder Säureamids, vorzugsweise Säureamids, sein. Insbesondere kann das Lokalanästhetikum ausgewählt sein aus der Gruppe von Lokalanästhetika vom Estertyp und Lokalanästhetika vom Amidtyp, vorzugsweise Lokalanästhetika vom Amidtyp.

Insbesondere kann das Lokalanästhetikum ausgewählt sein aus der Gruppe von Benzocain, Procain, Tetracain, Lidocain, Etidocain, Prilocain, Mepivacain, Bupivacain und S-Ropivacin und deren Salzen und Estern sowie deren Kombinationen und Mischungen, insbesondere Lidocain und dessen Salzen und Estern, vorzugsweise Lidocainhydrochlorid.

Vorliegend kann in Bezug auf die vorgenannten Aspekte das Lokalanästhetikum ausgewählt sein aus der Gruppe von Lidocain (2-Diethylamino-N-(2,6-dimethylphenyl)acetamid) und dessen Salzen und Estern, bevorzugt Lidocainhydrochlorid. Das Lokalanästhetikum kann somit insbesondere Lidocain und/oder dessen Salze und/oder Ester, bevorzugt Lidocainhydrochlorid, sein.

Im Hinblick auf die vorgenannten Aspekte kann es vorgesehen sein, dass das Octenidin und/oder dessen Salze und/oder Ester, vorzugsweise Octenidindihydrochlorid, gemeinsam mit mindestens einem Antiphlogistikum, insbesondere Benzydamin und/oder dessen Salzen und/oder Estern, bevorzugt Benzydaminhydrochlorid, eingesetzt und/oder verwendet wird.

Weiterhin kann gemäß den vorgenannten Aspekten das Octenidin und/oder dessen Salze und/oder Ester, vorzugsweise Octenidindihydrochlorid, gemeinsam mit mindestens einem nichtsteroidalen Antiphlogistikum (NSAR-Wirkstoff), insbesondere ausgewählt aus der Gruppe von Flurbiprofen, Ibuprofen, Dexibuprofen, Naproxen, Ketoprofen, Dexketoprofen, Tiaprofensäure, Diclofenac und Acetylsalicylsäure und deren Salzen und Estern sowie deren Kombinationen und Mischungen, insbesondere Flurbiprofen und dessen Salzen und Estern, vorzugsweise Flurbiprofen, eingesetzt bzw. verwendet werden.

Insbesondere kann das Octenidin und/oder dessen Salze und/oder Ester, vorzugsweise Octenidindihydrochlorid, gemeinsam mit Flurbiprofen und/oder dessen Salzen und/oder Estern, bevorzugt Flurbiprofen, eingesetzt und/oder verwendet werden.

Gemäß den drei unmittelbar vorgenannten Aspekten kann es gleichermaßen im Allgemeinen vorgesehen sein, dass das Octenidin und/oder dessen Salze und/oder Ester, vorzugsweise Octenidindihydrochlorid, mit mindestens einem Säuerungsmittel, insbesondere in Form einer organischen Säure oder deren Salze oder Ester, bevorzugt Weinsäure und/oder Citronensäure, bevorzugt Weinsäure, eingesetzt und/oder verwendet wird.

Zudem kann das Octenidin und/oder dessen Salze und/oder Ester, vorzugsweise Octenidindihydrochlorid, mit mindestens einem Anisöl, insbesondere Sternanisöl, eingesetzt und/oder verwendet werden.

Weiterhin kann das Octenidin und/oder dessen Salze und/oder Ester, vorzugsweise Octenidindihydrochlorid, mit mindestens einem ätherischen Öl, insbesondere Pfefferminzöl, eingesetzt und/oder verwendet werden.

Darüber hinaus kann das Octenidin und/oder dessen Salze und/oder Ester, vorzugsweise Octenidindihydrochlorid, mit mindestens einem Kaffee-Extrakt und/oder Tee-Extrakt, insbesondere Kaffee-Extrakt, eingesetzt und/oder verwendet werden.

Gemäß den drei unmittelbar vorgenannten Aspekten kann das Octenidin und/oder dessen Salze und/oder Ester, vorzugsweise Octenidindihydrochlorid, mit Propylenglykol eingesetzt und/oder verwendet werden.

Weiterhin kann das Octenidin und/oder dessen Salze und/oder Ester, vorzugsweise Octenidindihydrochlorid, mit mindestens einer Schleimdroge und/oder deren Extrakt eingesetzt und/oder verwendet werden. Dabei kann wobei die Schleimdroge bzw. deren Extrakt ausgewählt sein aus der Gruppe von Isländisch Moos (*Lichen islandicus*), Eibisch (*Althaea officinalis L.*), Spitzwegerich (*Plantago lanceolata L.*), Malve (*Malva sylvestris L.* und *M. neglecta WALLR.* und andere), Boxhornklee (*Trigonella foenum-graecum L.*), Salep und Quitte (*Cydonia oblonga MILL.*) sowie deren Kombinationen und Mischungen, bevorzugt Isländisch Moos (*Lichen islandicus*) und/ oder Eibisch *(Althaea officinalis L.*)*.*

Im Hinblick auf die drei unmittelbar vorgenannten Aspekte kann es zudem vorgesehen sein, dass das Octenidin und/oder dessen Salze und/oder Ester, vorzugsweise Octenidindihydrochlorid, nicht gemeinsam mit und/oder in Abwesenheit von Phenoxyethanol (2-Phenoxyethanol) eingesetzt und/oder verwendet wird.

Zudem kann es vorgesehen sein, dass das Octenidin und/oder dessen Salze und/oder Ester, vorzugsweise Octenidindihydrochlorid, nicht gemeinsam mit und/oder in Abwesenheit von n-Propanol (Propan-1-ol) und/oder nicht gemeinsam mit Isopropanol, insbesondere nicht gemeinsam mit einem primären Alkohol, eingesetzt und/oder verwendet wird.

Weiterführend kann es auch für die drei unmittelbar vorgenannten Aspekte vorgesehen sein, dass das Octenidin und/oder dessen Salze und/oder Ester, vorzugsweise Octenidindihydrochlorid, mit einer Tagesdosis im Bereich von 0,15 mg/diem bis 120 mg/diem, insbesondere im Bereich von 0,15 mg/diem bis 120 mg/diem, vorzugsweise im Bereich von 0,3 mg/diem bis 100 mg/diem, bevorzugt im Bereich von 1,5 mg/diem bis 60 mg/diem, besonders bevorzugt im Bereich von 2,25 mg/diem bis 120 mg/diem, weiter bevorzugt im Bereich von 2,5 mg/diem bis 40 mg/diem, verabreicht wird.

Insbesondere kann das Octenidin und/oder dessen Salze und/oder Ester, vorzugsweise Octenidindihydrochlorid, zur Verabreichung einer Tagesdosis im Bereich von 0,15 mg/diem bis 120 mg/diem, insbesondere im Bereich von 0,15 mg/diem bis 120 mg/diem, vorzugsweise im Bereich von 0,3 mg/diem bis 100 mg/diem, bevorzugt im Bereich von 1,5 mg/diem bis 60 mg/diem, besonders bevorzugt im Bereich von 2,25 mg/diem bis 120 mg/diem, weiter bevorzugt im Bereich von 2,5 mg/diem bis 40 mg/diem, hergerichtet sein.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, jedoch ohne die vorliegende Erfindung hierauf beschränken zu wollen.

### AUSFÜHRUNGSBEISPIELE:

### 1. Herstellungsbeispiele:

**Herstellung erfindungsgemäß einsetzbarer Lutschtabletten auf Hartkaramellenbasis**

Es werden verschiedene erfindungsgemäß einsetzbare Lutschtabletten auf Hartkaramellenbasis hergestellt. Für die zuckerhaltige Variante können z. B. Saccharose- und/oder Glucosesirup eingesetzt werden, während für die zuckerfreie Variante Zuckeraustauschstoffe, insbesondere Zuckeralkohole, wie z. B. Maltit (Maltitol) oder Isomalt bzw. Isomaltit (Palatinit^{®}), als Lutschtablettenbasis bzw. Hartkaramellenbasis (Matrix) eingesetzt werden können, wobei sowohl bei der zuckerhaltigen als auch bei der zuckerfreien Variante gegebenenfalls weitere Wirk- und/oder Inhaltsstoffe (z. B. Lokalanästhetika, Schleimdrogen bzw. deren Extrakte, Verarbeitungshilfsmittel, Aromen und Aromastoffe, Geschmacksstoffe, Süßstoffe und Süßungsmittel, Säuerungsmittel, Stabilisatoren, Antiseptika, Farbstoffe etc.) zugesetzt sein können. Die entsprechenden Wirk- und Inhaltsstoffe werden in die Matrix eingearbeitet. Die Herstellung der Lutschtabletten bzw. Hartkaramellen wird dabei wie folgt durchgeführt:
Der Zucker bzw. die Zuckerersatzstoffe/Zuckeraustauschstoffe werden in Wasser gelöst und anschließend bei Temperaturen zwischen 120 und 140 °C gekocht und vakuumiert. Dieser heißen Masse werden der Wirkstoff (Octenidindihydrochlorid) sowie gegebenenfalls weitere Wirk- und/oder Inhaltsstoffe, wie zuvor genannt, in fester oder flüssiger Form zudosiert. Nach kontrolliertem Abkühlen auf Temperaturen unterhalb von 75 °C werden die Hartkaramellen in der gewünschten Form geprägt und nachfolgend verpackt.

Auf diese Weise werden verschiedene Rezepturen erfindungsgemäß einsetzbarer Lutschtabletten mit variablen Mengen an Inhaltsstoffen hergestellt:

### Rezeptur 1: Lutschtabletten, zuckerfrei (Isomalt)

| | |
|---|---|
| Octenidindihydrochlorid: | 0,05 - 2,0 Gew.-% |
| Isomalt: | 93,0 - 99,95 Gew.-% |
| Säuerungsmittel, Farbstoffe, Süßstoffe und Aromen: | 0 - 5,0 Gew.-% |
| Summe: | 1.000 - 4.000 mg |

### Rezeptur 2: Lutschtablette, zuckerfrei (Maltitol)

| | |
|---|---|
| Octenidindihydrochlorid: | 0,05 - 2,0 Gew.-% |
| Maltitol: | 93,0 - 99,95 Gew.-% |
| Säuerungsmittel, Farbstoffe, Süßstoffe und Aromen: | 0 - 5,0 Gew.-% |
| Summe: | 1.000 - 4.000 mg |

### Rezeptur 3: Lutschtabletten, zuckerfrei (Maltitol/Isomalt)

| | |
|---|---|
| Octenidindihydrochlorid: | 0,05 - 2,0 Gew.-% |
| Maltitol/Isomalt 1 : 0,10 - 4: | 93,0 - 99,95 Gew.-% (Trockensubstanz) |
| Säuerungsmittel, Farbstoffe, Süßstoffe und Aromen: | 0 - 5,0 Gew.-% |
| Summe: | 1.000 - 4.000 mg |

### Rezeptur 4: Lutschtabletten, zuckerhaltig (Saccharose/Glucose-Sirup)

| | |
|---|---|
| Octenidindihydrochlorid: | 0,05 - 2,0 Gew.-% |
| Saccharose/Glucose-Sirup 1 : 0,5 - 4: | 93,0 - 99,95 Gew.-% (Trockensubstanz) |
| Säuerungsmittel, Farbstoffe, Süßstoffe und Aromen: | 0 - 5,0 Gew.-% |
| Summe: | 1.000 - 4.000 mg |

In entsprechender Weise wird folgende Rezeptur erfindungsgemäß einsetzbarer Lutschtabletten hergestellt:

### Rezeptur 5: Lutschtabletten, zuckerfrei (Isomalt)

| | |
|---|---|
| Octenidindihydrochlorid: | 0,05 - 2,0 Gew-% |
| Isomalt (Trockensubstanz): | 93,0 - 99,95 Gew.-% |
| Säuerungsmittel, Farbstoffe, Aroma: | 0 - 1,5 Gew.-% |
| Weinsäure: | 0 - 2,0 Gew.-% |
| Sternanisöl: | 0 - 1,0 Gew.-% |
| Pfefferminzöl: | 0 - 0,5 Gew.-% |
| Summe: | 1.000 - 4.000 mg |

### 2. Wirksamkeitsuntersuchungen

### (i) Beispiel 1:

Die antivirale Wirksamkeit gegenüber SARS-CoV-2 einer erfindungsgemäß verwendbaren Octenidindihydrochlorid-Zusammensetzungen wird im Vergleich zu Placebo an Patienten mit vorliegender SARS-CoV-2-Infektion untersucht.

Hierzu wird eine erfindungsgemäß verwendbare Zusammensetzung mit Octenidindihydrochlorid auf Basis der zuvor beschriebenen Rezeptur 1 eingesetzt, und zwar als feste Dosierungsform in Form einer als Hartkaramelle ausgebildeten Lutschtablette (nachfolgend auch als "Octenidindihydrochlorid-Lutschtablette" bezeichnet), wobei eine jeweilige Dosiereinheit in Form der Lutschtablette einen Gehalt an Octenidindihydrochlorid von 2,6 mg und einen Isomaltgehalt von 2,57 g (entsprechend etwa 6 kcal bzw. etwa 26 kJ) aufweist.

Bei der hierzu korrespondierenden Placebo-Zusammensetzung, die ebenfalls als feste Dosierungsform in Form einer als Hartkaramelle ausgebildeten Lutschtablette vorliegt (nachfolgend auch als "Placebo-Lutschtablette" bezeichnet), ist der Wirkstoff Octenidindihydrochlorid durch eine entsprechende Menge an Isomalt ersetzt.

Der Nachweis von SARS-CoV-2 erfolgt bei den jeweiligen Patienten mittels eines entsprechenden PCR-Tests (*Polymerase Chain Reaction* bzw. Polymerase-Kettenreaktion), wobei hierfür Proben der Patienten auf Basis pharyngealer Abstriche eingesetzt werden. Anhand des PCR-Tests wird jeweils der sogenannte Ct-Wert (*Cycle threshold*) ermittelt, wobei der CT-Wert auf die erforderliche Anzahl der zum Nachweis von SARS-CoV-2 durchgeführten Messzyklen zurückgeht.

Ein kleiner Ct-Wert bedeutet somit ein hohe Virenlast, da relativ wenig Messzyklen durchgeführt werden müssen, um das Virus nachzuweisen. Ein hoher Ct-Wert bedeutet ein geringe Virenlast, da relativ viele Messzyklen durchgeführt werden müssen, um das Virus nachzuweisen. Ein negativer Befund (d.h. kein Nachweis von SARS-CoV-2) ist vorliegend bei einem Ct-Wert von 38 gegeben.

Zunächst wird bei den Patienten ein erster PCR-Test vor Verabreichung der jeweiligen Lutschtablette durchgeführt und der entsprechende Ct-Wert ermittelt (in den nachfolgenden Tabellen 1 und 2 unter "Ct0" angeführt).

Die Patienten erhalten nachfolgend entweder eine Octenidindihydrochlorid-Lutschtablette ("Octenidindihydrochlorid-Gruppe") oder aber eine Placebo-Lutschtablette ("Placebo-Gruppe"), wobei pro Patient eine Lutschtablette verabreicht wird. Die Lutschdauer beträgt 15 min.

Um Lutsch- bzw. Verdünnungseffekte zu vermeiden bzw. auszuschließen, wird der nachfolgende zweite PCR-Test erst 15 min nach Beendigung des Lutschens der verabreichten Zusammensetzung (d. h. 30 min nach Lutschbeginn) durchgeführt und der entsprechende Ct-Wert ermittelt (in den nachfolgenden Tabellen 1 und 2 unter "Ct1" angegeben).

Die nachfolgende **Tabelle 1** zeigt die entsprechenden Ergebnisse, welche für die Octenidindihydrochlorid-Gruppe erhalten werden:

| **Patient / Octenidindihydrochlorid-Lutschtablette** | **Ct0** | **Ct1** |
|---|---|---|
| O1 | 30,87 | 33,04 |
| O2 | 17,77 | 21,37 |
| O3 | 28,77 | 30,65 |
| O4 | 37,06 | negativ |
| O5 | 34,16 | 36,04 |
| O6 | 27,31 | negativ |
| O7 | 17,83 | 19,40 |
| O8 | 26,74 | 34,09 |
| O9 | 22,65 | 29,75 |
| O10 | 21,58 | 28,85 |
| O11 | 34,46 | 35,16 |
| O12 | 21,64 | 22,50 |
| O13 | 32,03 | 33,88 |
| O14 | 29,65 | 31,35 |
| O15 | 28,30 | 30,14 |
| O16 | 27,16 | 29,19 |
| O17 | 21,86 | 28,79 |
| O18 | 21,46 | 29,54 |
| O19 | 23,89 | 30,19 |
| O20 | 24,77 | 29,78 |

Darüber hinaus zeigt die nachfolgende **Tabelle 2** die für die Placebo-Gruppe erhaltenen Ergebnisse:

| **Patient / Placebo-Lutschtablette** | **Ct0** | **Ct1** |
|---|---|---|
| P1 | 35,69 | 34,17 |
| P2 | 25,16 | 25,11 |
| P3 | 19,45 | 20,17 |

Die in Tabelle 1 und 2 dargestellten Ergebnisse zeigen, dass unter Verabreichung erfindungsgemäß einsetzbarer Octenidindihydrochlorid-Lutschtabletten ein deutlicher antiviraler Wirkeffekt in Bezug auf SARS-CoV2 vorliegt. So kann für die mit der Octenidindihydrochlorid-Lutschtablette behandelten Patienten gegenüber der Placebo-Gruppe eine deutliche Zunahme des in den PCR-Untersuchungen ermittelten Ct-Wertes beobachtet werden, und dies bereits nach einmaliger Verabreichung der Zusammensetzung. Die Ct1-Werte zeigen im Vergleich zu den Ct0-Werten das Vorliegen einer deutlich verringerten Virenlast nach Verabreichung der Octenidindihydrochlorid-Lutschtablette und auch im Vergleich zu der Placebo-Gruppe.

Die obigen Untersuchungen zeigen somit den antiviralen Effekt in Bezug auf das Corona-Virus SARS-CoV2 der erfindungsgemäß einsetzbaren Zusammensetzung auf Basis der Octenidindihydrochlorid-Lutschtablette.

### (ii) Beispiel 2:

Darüber hinaus wird die antivirale Wirksamkeit einer erfindungsgemäß einsetzbaren Zusammensetzung in Form der in Beispiel 1 beschriebenen Octenidindihydrochlorid-Lutschtablette an weiteren Patienten untersucht. Die Patienten weisen im Hinblick auf SARS-CoV2 vor Verabreichung der Lutschtabletten einen positiven PCR-Test auf (in der nachfolgenden Tabelle 3 unter "Ct0" angegeben).

Die Patienten erhalten insgesamt jeweils drei Octenidindihydrochlorid-Lutschtabletten in definierter zeitlicher Abfolge. Die Lutschzeit beträgt für eine jeweilige Tablette 15 Minuten, wobei jeweils nach jeweils weiteren 15 Minuten ein PCR-Test durchgeführt wird. Der PCR-Test nach Verabreichung der ersten Octenidindihydrochlorid-Lutschtablette wird somit 30 Minuten nach erfolgter Verabreichung durchgeführt (in der nachfolgenden Tabelle 3 unter "Ct1" angeführt).

Die zweite Octenidindihydrochlorid-Lutschtablette wird 2,5 Stunden nach Verabreichung der ersten Lutschtablette verabreicht, wobei der diesbezügliche PCR-Test 3 Stunden nach Verabreichung der ersten Lutschtablette und 30 Minuten nach Verabreichung der zweiten Lutsch-tablette durchgeführt wird (in der nachfolgenden Tabelle 3 unter "Ct2" angeführt).

Zudem wird die dritte Octenidindihydrochlorid-Lutschtablette 5,5 Stunden nach Verabreichung der ersten Lutschtablette verabreicht, wobei der diesbezügliche PCR-Test gleichermaßen 30 Minuten nach Verabreichung der dritten Lutschtablette und somit 6 Stunden nach Verabreichung der ersten Lutschtablette durchgeführt wird (in der nachfolgenden Tabelle 3 unter "Ct3" angeführt).

Die nachfolgende **Tabelle 3** zeigt die diesbezüglich ermittelten Ergebnisse:

| **Patient / Octenidindihydrochlorid-Lutschtablette** | **Ct0** | **Ct1** | **Ct2** | **Ct3** |
|---|---|---|---|---|
| Oct1 | 21,58 | 22,39 | 28,46 | 30,15 |
| Oct2 | 26,35 | 34,43 | negativ | negativ |
| Oct3 | 24,18 | 29,68 | 31,49 | 34,72 |
| Oct4 | 27,90 | 31,72 | 31,10 | 34,03 |

In analoger Weise erfolgt die Verabreichung der in Beispiel 1 beschrieben Placebo-Lutschtabletten an einem weiteren Patienten.

Die nachfolgende **Tabelle 4** zeigt die ermittelten Ergebnisse:

| **Patient / Placebo-Lutschtablette** | **Ct0** | **Ct1** | **Ct2** | **Ct3** |
|---|---|---|---|---|
| Pla1 | 24,08 | 23,24 | 24,10 | 23,86 |

Die Ergebnisse zeigen, dass im zeitlichen Verlauf mit der aufeinanderfolgenden Verabreichung mehrerer Lutschtabletten unter Verwendung von Octenidindihydrochlorid als Wirksubstanz eine deutliche und nachhaltige Zuname des jeweiligen Ct-Werts vorliegt, einhergehend mit einer geringeren Virenlast, und zwar auch im Vergleich zu Placebo.

Somit zeigen auch die obigen Untersuchungen die erfindungsgemäß aufgefundene antivirale Wirkung von Octenidindihydrochlorid im Rahmen seiner topischen Anwendung.

### (iii) Beispiel 3:

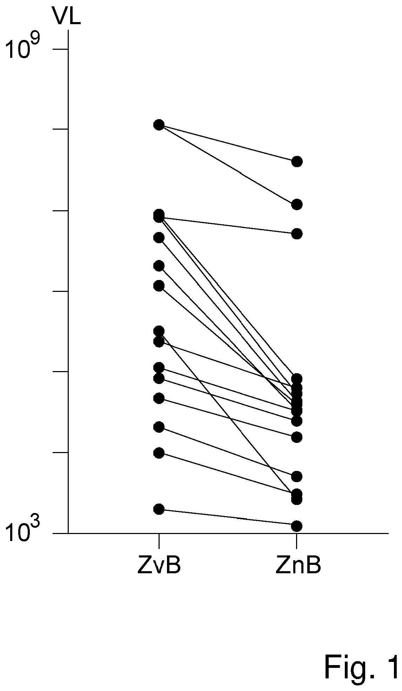

Darüber hinaus wird für Patienten mit positiven SARS-CoV2-Befunden die Virenlast auf Basis von Abstrichen aus dem Mund- bzw. Rachenraum bestimmt. Die ermittelten Ergebnisse sind in der einzigen Figur (Fig. 1) dargestellt.

Die Patienten werden in diesem Zusammenhang mit einer erfindungsgemäß einsetzbaren Zusammensetzung in Form der in Beispiel 1 beschriebenen Octenidindihydrochlorid-Lutschtabletten behandelt.

Die grafische Darstellung gemäß der einzigen Figur (Fig. 1) zeigt dabei den Zustand bzw. Wert der Virenlast ("VL") vor der Behandlung mit der Zusammensetzung ("ZvB") sowie nach der Behandlung ("ZnB"). Die Bestimmung der Virenlast bezieht sich dabei auf eine jeweilige Probe, welche 30 Minuten nach Verabreichung einer Octenidindihydrochlorid-Lutschtablette entnommen wird, wobei der Lutschzeitraum 15 Minuten beträgt, so dass etwaige Lutsch- bzw. Verdünnungseffekte vermieden bzw. ausgeschlossen werden.

Die einzige Figur (Fig. 1) zeigt dabei insgesamt die deutliche Verringerung der Virenlast nach Verabreichung der Octenidindihydrochlorid-Lutschtablette.

Insgesamt zeigen die obigen Untersuchungen der Anmelderin somit, dass der Wirkstoff Octenidin bzw. dessen Salze bzw. Ester, insbesondere Octenidindihydrochlorid, imstande ist, bei der prophylaktischen bzw. therapeutischen Behandlung von durch SARS-CoV2 hervorgerufenen Viruserkrankungen, insbesondere COVID-19, die Virenlast aufgrund der überraschend gefundenen antiviralen Wirkung bei topischer Anwendung im Mund- bzw. Rachenraum zu verringern bzw. abzuschwächen. Diese antivirale Wirkung von Octenidin bzw. Octenidindihydrochlorid ist vollkommen unerwartet.

Insgesamt eignet sich somit Octenidin bzw. dessen Salze und/oder Ester, insbesondere Octenidindihydrochlorid, auf Basis der überraschend aufgefundenen Erkenntnisse der Anmelderin in effizienter Weise zur Verwendung bei der prophylaktischen bzw. therapeutischen topischen Behandlung von durch Corona-Viren, insbesondere SARS-CoV2 hervorgerufenen Viruserkrankungen, insbesondere COVID-19.

## Patentansprüche

1. Zusammensetzung, insbesondere pharmazeutische Zusammensetzung, zur Verwendung bei der prophylaktischen und/oder therapeutischen topischen Behandlung von durch Corona-Viren hervorgerufenen Viruserkrankungen und zur topischen Anwendung auf die Schleimhäute des Mund-, Rachen- und/oder Nasenraums,
wobei die Zusammensetzung als antiviralen Wirkstoff Octenidin und/oder dessen Salze und/oder Ester, vorzugsweise Octenidindihydrochlorid, enthält,
wobei die Zusammensetzung als feste Dosierungsform in Form einer Lutschtablette vorliegt und/oder ausgebildet ist, wobei die Lutsch-tablette ein Gesamtgewicht im Bereich von 0,5 g bis 7 g, insbesondere im Bereich von 1 g bis 6 g, vorzugsweise im Bereich von 2 g bis 5 g, aufweist, enthaltend je Lutschtablette:
- Octenidin und/oder dessen Salze und/oder Ester, vorzugsweise Octenidindihydrochlorid, in einer Menge im Bereich von 0,05 mg bis 15 mg, insbesondere im Bereich von 0,1 mg bis 12,5 mg, vorzugsweise im Bereich von 0,5 mg bis 10 mg, bevorzugt im Bereich von 0,75 mg bis 7,5 mg, besonders bevorzugt im Bereich von 1 mg bis 5 mg;
- gegebenenfalls mindestens ein Lokalanästhetikum, insbesondere Lidocain, bevorzugt Lidocainhydrochlorid, in einer Menge im Bereich von 0,1 mg bis 50 mg, insbesondere im Bereich von 1 mg bis 20 mg, vorzugsweise im Bereich von 3 mg bis 15 mg, bevorzugt im Bereich von 4 mg bis 10 mg;
- gegebenenfalls mindestens ein Säuerungsmittel in Form einer organischen Säure oder deren Salze oder Ester, bevorzugt Weinsäure und/oder Citronensäure, in einer Menge im Bereich von 0,5 mg bis 100 mg, insbesondere im Bereich von 1 mg bis 50 mg, vorzugsweise im Bereich von 2 mg bis 20 mg;
- gegebenenfalls mindestens ein Anisöl, insbesondere Sternanisöl, in einer Menge im Bereich von 0,2 mg bis 80 mg, insbesondere im Bereich von 0,5 mg bis 40 mg, vorzugsweise im Bereich von 0,75 mg bis 15 mg;
- gegebenenfalls mindestens ein ätherisches Öl, insbesondere Pfefferminzöl, in einer Menge im Bereich von 0,01 mg bis 5 mg, insbesondere im Bereich von 0,05 mg bis 3 mg, vorzugsweise im Bereich von 0,1 mg bis 1 mg;
- gegebenenfalls mindestens ein Kaffee-Extrakt und/oder Tee-Extrakt, insbesondere Kaffee-Extrakt, in einer Menge im Bereich von 0,05 mg bis 100 mg, insbesondere im Bereich von 0,5 mg bis 50 mg, vorzugsweise im Bereich von 1 mg bis 20 mg;
- gegebenenfalls Propylenglykol in einer Menge im Bereich von 0,05 mg bis 50 mg, insbesondere im Bereich von 0,5 mg bis 20 mg, vorzugsweise im Bereich von 1 mg bis 10 mg;
- gegebenenfalls mindestens eine Schleimdroge und/oder deren Extrakt in einer Menge im Bereich von 0,5 mg bis 200 mg, insbesondere im Bereich von 1 mg bis 100 mg, vorzugsweise im Bereich von 5 mg bis 50 mg;
- gegebenenfalls mindestens einen weiteren Wirk- und/oder Inhaltsstoff, insbesondere pharmazeutischen Zusatz- und/oder Hilfsstoff, insbesondere ausgewählt aus der Gruppe von Verarbeitungshilfsstoffen, Stabilisatoren, Emulgatoren, Antioxidantien, Konservierungsstoffen, Feuchthaltemitteln, Süßstoffen und Süßungsmitteln, pH-Stellmitteln, pH-Puffersubstanzen, Verdickungsmitteln, Aromastoffen, Antiseptika, Färbe-, Puffer-, Riech-, Duft-, Streck-, Binde-, Netz- und/oder Konservierungsstoffen sowie deren Kombinationen und Mischungen, in einer Menge im Bereich von 0,1 mg bis 500 mg, insbesondere im Bereich von 0,5 mg bis 250 mg, vorzugsweise im Bereich von 1 mg bis 100 mg;
- mindestens einen Zucker und/oder mindestens einen Zuckeraustauschstoff als Exzipient; wobei der Zucker ausgewählt ist aus der Gruppe von Saccharose, Glucose, insbesondere Dextrose, und Fructose und/oder wobei der Zuckeraustauschstoff ausgewählt ist aus Zuckeralkoholen, vorzugsweise aus der Gruppe von Mannit, Xylit, Sorbit, Isomalt, Maltitsirup, Lactit, Leucrose, Fructooligosacchariden, Glucanen, Polyglucose sowie deren Kombinationen und Mischungen, besonders bevorzugt Isomalt und/oder Mannit sowie deren Kombinationen und Mischungen, ganz besonders bevorzugt Isomalt; und in Mengen zur Bereitstellung und/oder Erhalt des Gesamtgewichts der Lutschtablette,
wobei die Lutschtablette zumindest im Wesentlichen frei ist von Phenoxyethanol.

2. Zusammensetzung zur Verwendung nach Anspruch 1,
wobei die Zusammensetzung das Octenidin und/oder dessen Salze und/oder Ester in Form eines Hydrochloridsalzes, vorzugsweise Octenidindihydrochlorid, enthält.

3. Zusammensetzung zur Verwendung nach Anspruch 1 oder 2,
wobei das Lokalanästhetikum ausgewählt ist aus der Gruppe von Lidocain und dessen Salzen und Estern, bevorzugt Lidocainhydrochlorid.

4. Zusammensetzung zur Verwendung nach einem der vorangehenden Ansprüche,
wobei die Zusammensetzung mindestens ein Antiphlogistikum, insbesondere Benzydamin und/oder dessen Salze und/oder Ester, bevorzugt Benzydaminhydrochlorid, enthält.

5. Zusammensetzung zur Verwendung nach einem der vorangehenden Ansprüche,
wobei die Zusammensetzung mindestens ein nichtsteroidales Antiphlogistikum enthält, insbesondere wobei das nichtsteroidale Antiphlogistikum ausgewählt ist aus der Gruppe von Flurbiprofen, Ibuprofen, Dexibuprofen, Naproxen, Ketoprofen, Dexketoprofen, Tiaprofensäure, Diclofenac und Acetylsalicylsäure und deren Salzen und Estern sowie deren Kombinationen und Mischungen, insbesondere Flurbiprofen und dessen Salzen und Estern, vorzugsweise Flurbiprofen; und/oder
wobei die Zusammensetzung Flurbiprofen und/oder dessen Salze und/oder Ester, bevorzugt Flurbiprofen, enthält.

6. Zusammensetzung zur Verwendung nach einem der vorangehenden Ansprüche bei der prophylaktischen und/oder therapeutischen topischen Behandlung von durch SARS-CoV-2 hervorgerufenen Viruserkrankungen, bevorzugt von COVID-19.

7. Zusammensetzung zur Verwendung nach einem der vorangehenden Ansprüche,
wobei die Zusammensetzung zumindest im Wesentlichen frei ist von n-Propanol und/oder zumindest im Wesentlichen frei ist von Isopropanol, insbesondere zumindest im Wesentlichen frei ist von einem primären Alkohol.

8. Zusammensetzung zur Verwendung nach einem der vorangehenden Ansprüche,
wobei die Zusammensetzung das Antiphlogistikum, insbesondere Benzydamin und/oder dessen Salze und/oder Ester, bevorzugt Benzydaminhydrochlorid, in einer Menge im Bereich von 0,1 mg bis 50 mg, insbesondere im Bereich von 0,5 mg bis 20 mg, vorzugsweise im Bereich von 1 mg bis 15 mg, bevorzugt im Bereich von 2 mg bis 10 mg, bezogen auf eine Darreichungseinheit der Zusammensetzung, enthält.

9. Zusammensetzung zur Verwendung nach einem der vorangehenden Ansprüche,
wobei die Zusammensetzung das nichtsteroidale Antiphlogistikum, insbesondere Flurbiprofen und/oder dessen Salze und/oder Ester, bevorzugt Flurbiprofen, in einer Menge im Bereich von 0,1 mg bis 50 mg, insbesondere im Bereich von 0,5 mg bis 20 mg, vorzugsweise im Bereich von 1 mg bis 15 mg, bevorzugt im Bereich von 2 mg bis 10 mg, bezogen auf eine Darreichungseinheit der Zusammensetzung, enthält.

10. Zusammensetzung zur Verwendung nach einem der vorangehenden Ansprüche,
wobei das Octenidin und/oder dessen Salze und/oder Ester, vorzugsweise Octenidindihydrochlorid, mit einer Tagesdosis im Bereich von 0,15 mg/diem bis 120 mg/diem, insbesondere im Bereich von 0,15 mg/diem bis 120 mg/diem, vorzugsweise im Bereich von 0,3 mg/diem bis 100 mg/diem, bevorzugt im Bereich von 1,5 mg/diem bis 60 mg/diem, besonders bevorzugt im Bereich von 2,25 mg/diem bis 120 mg/diem, weiter bevorzugt im Bereich von 2,5 mg/diem bis 40 mg/diem, verabreicht wird; und/oder
wobei die Zusammensetzung zur Verabreichung von Octenidin und/oder dessen Salzen und/oder Estern, vorzugsweise Octenidindihydrochlorid, in einer Tagesdosis im Bereich von 0,15 mg/diem bis 120 mg/diem, insbesondere im Bereich von 0,15 mg/diem bis 120 mg/diem, vorzugsweise im Bereich von 0,3 mg/diem bis 100 mg/diem, bevorzugt im Bereich von 1,5 mg/diem bis 60 mg/diem, besonders bevorzugt im Bereich von 2,25 mg/diem bis 120 mg/diem, weiter bevorzugt im Bereich von 2,5 mg/diem bis 40 mg/diem, hergerichtet ist.

11. Zusammensetzung zur Verwendung nach einem der vorangehenden Ansprüche,
wobei die Zusammensetzung als Hartkaramelle vorliegt und/oder ausgebildet ist; und/oder
wobei die Zusammensetzung als Lutschtablette vorliegt und/oder ausgebildet ist, so dass die Zusammensetzung eine therapeutisch wirksame Menge an Wirk- und/oder Inhaltsstoffen, insbesondere Octenidin und/oder dessen Salze und/oder Ester, vorzugsweise Octenidindihydrochlorid, beim Lutschen freisetzt, wenn die Zusammensetzung, insbesondere die feste Dosierungsform, im Mund- und Rachenraum eines Patienten verabreicht und gelutscht wird.

12. Zusammensetzung zur Verwendung nach einem der vorangehenden Ansprüche,
wobei die Zusammensetzung, insbesondere die feste Dosierungsform, die Wirk- und/oder Inhaltsstoffe in einer festen Matrix und/oder Masse, insbesondere in einer Matrix und/oder Masse auf Basis von Zuckern und/oder Zuckeraustauschstoffen aufweist.

13. Zusammensetzung zur Verwendung nach einem der vorangehenden Ansprüche,
wobei die Zusammensetzung, insbesondere die feste Dosierungsform, eine Restfeuchte im Bereich von 0,1 Gew.-% bis 10 Gew.-%, insbesondere im Bereich von 0,5 Gew.-% bis 5 Gew.-%, vorzugsweise im Bereich von 1 Gew.-% bis 3 Gew.-%, bezogen Zusammensetzung, insbesondere die feste Dosierungsform, aufweist.

14. Zusammensetzung zur Verwendung nach einem der vorangehenden Ansprüche,
wobei die Zusammensetzung zur Expositionsprophylaxe, insbesondere Postexpositionsprophylaxe und/oder zur Präexpositionsprophylaxe, gegenüber Corona-Viren, vorzugsweise SARS-CoV-2, eingesetzt wird; und/oder
wobei die Zusammensetzung zur Infektionsprophylaxe gegenüber Corona-Viren, vorzugsweise SARS-CoV-2, eingesetzt wird; und/oder
wobei die Zusammensetzung zur Verringerung der Virenlast von Corona-Viren, vorzugsweise SARS-CoV-2, bevorzugt zur Verringerung der Virenlast im Mund-, Rachen- und/oder Nasenraum, eingesetzt wird; und/oder
wobei die Zusammensetzung im präsymptomatischen Stadium und/oder postsymptomatischen Stadium, insbesondere im präsymptomatischen Stadium, von durch Corona-Viren, vorzugsweise SARS-CoV-2, hervorgerufenen Viruserkrankungen, bevorzugt von COVID-19, eingesetzt wird; und/oder
wobei die Zusammensetzung bei asymptomatischen Verläufen von durch Corona-Viren, vorzugsweise SARS-CoV-2, hervorgerufenen Viruserkrankungen, bevorzugt von COVID-19, eingesetzt wird; und/oder
wobei die Zusammensetzung bei symptomatischen Verläufen von durch Corona-Viren, vorzugsweise SARS-CoV-2, hervorgerufenen Viruserkrankungen, bevorzugt von COVID-19, eingesetzt wird.

15. Zusammensetzung zur Verwendung nach einem der vorangehenden Ansprüche,
wobei die Zusammensetzung, insbesondere das Octenidin und/oder dessen Salze und/oder Ester, vorzugsweise Octenidindihydrochlorid, die Corona-Viren, vorzugsweise SARS-CoV-2, inaktiviert und/oder abtötet; und/oder
wobei die Zusammensetzung, insbesondere das Octenidin und/oder dessen Salze und/oder Ester, vorzugsweise Octenidindihydrochlorid, die Vermehrung und/oder Replikation der Corona-Viren, vorzugsweise SARS-CoV-2, hemmt und/oder vermindert und/oder unterbindet.

## Claims

1. Composition, in particular pharmaceutical composition, for use in the prophylactic and/or therapeutic topical treatment of viral diseases caused by coronaviruses and for topical application to the mucous membranes of the oral, pharyngeal, and/or nasal cavity, wherein the composition comprises octenidine and/or its salts and/or esters, preferably octenidine dihydrochloride, as the antiviral active ingredient,
wherein the composition is present and/or formulated as a solid dosage form in the form of a lozenge, wherein the lozenge has a total weight in the range from 0.5 g to 7 g, in particular in the range from 1 g to 6 g, preferably in the range from 2 g to 5 g, comprising per lozenge:
- octenidine and/or its salts and/or esters, preferably octenidine dihydrochloride, in an amount ranging from 0.05 mg to 15 mg, in particular from 0.1 mg to 12.5 mg, preferably from 0.5 mg to 10 mg, more preferably from 0.75 mg to 7.5 mg, most preferably from 1 mg to 5 mg;
- optionally, at least one local anesthetic, in particular lidocaine, preferably lidocaine hydrochloride, in an amount ranging from 0.1 mg to 50 mg, in particular from 1 mg to 20 mg, preferably from 3 mg to 15 mg, more preferably from 4 mg to 10 mg;
- optionally, at least one acidifying agent in the form of an organic acid or its salts or esters, preferably tartaric acid and/or citric acid, in an amount ranging from 0.5 mg to 100 mg, in particular from 1 mg to 50 mg, preferably from 2 mg to 20 mg;
- optionally, at least one anise oil, in particular star anise oil, in an amount ranging from 0.2 mg to 80 mg, in particular from 0.5 mg to 40 mg, preferably from 0.75 mg to 15 mg;
- optionally, at least one essential oil, in particular peppermint oil, in an amount ranging from 0.01 mg to 5 mg, in particular from 0.05 mg to 3 mg, preferably from 0.1 mg to 1 mg;
- optionally, at least one coffee extract and/or tea extract, in particular coffee extract, in an amount ranging from 0.05 mg to 100 mg, in particular from 0.5 mg to 50 mg, preferably from 1 mg to 20 mg;
- optionally, propylene glycol in an amount ranging from 0.05 mg to 50 mg, in particular from 0.5 mg to 20 mg, preferably from 1 mg to 10 mg;
- optionally, at least one mucilaginous drug and/or its extract in an amount ranging from 0.5 mg to 200 mg, in particular from 1 mg to 100 mg, preferably from 5 mg to 50 mg;
- optionally, at least one further active ingredient and/or constituent, in particular a pharmaceutical additive and/or excipient, in particular selected from the group consisting of processing aids, stabilizers, emulsifiers, antioxidants, preservatives, humectants, sweeteners, pH adjusters, pH buffer substances, thickeners, flavoring agents, antiseptics, coloring agents, buffering agents, odorants, fragrances, diluents, binding agents, surfactants and/or preservatives, as well as combinations and mixtures thereof, in an amount ranging from 0.1 mg to 500 mg, in particular from 0.5 mg to 250 mg, preferably from 1 mg to 100 mg;
- at least one sugar and/or at least one sugar substitute as an excipient; wherein the sugar is selected from the group consisting of sucrose, glucose, in particular dextrose, and fructose, and/or wherein the sugar substitute is selected from sugar alcohols, preferably from the group consisting of mannitol, xylitol, sorbitol, isomalt, maltitol syrup, lactitol, leucrose, fructooligosaccharides, glucans, polyglucose, as well as combinations and mixtures thereof, more preferably isomalt and/or mannitol, as well as combinations and mixtures thereof, most preferably isomalt; and in amounts sufficient to provide and/or maintain the total weight of the lozenge,
wherein the lozenge is at least substantially free of phenoxyethanol.

2. Composition for the use according to claim 1,
wherein the composition comprises octenidine and/or its salts and/or esters in the form of a hydrochloride salt, preferably octenidine dihydrochloride.

3. Composition for the use according to claim 1 or 2,
wherein the local anesthetic is selected from the group consisting of lidocaine and its salts and esters, preferably lidocaine hydrochloride.

4. Composition for the use according to any of the preceding claims,
wherein the composition comprises at least one anti-inflammatory agent, in particular benzydamine and/or its salts and/or esters, preferably benzydamine hydrochloride.

5. Composition for the use according to any of the preceding claims,
wherein the composition comprises at least one nonsteroidal anti-inflammatory agent, in particular wherein the nonsteroidal anti-inflammatory agent is selected from the group consisting of flurbiprofen, ibuprofen, dexibuprofen, naproxen, ketoprofen, dexketoprofen, tiaprofenic acid, diclofenac and acetylsalicylic acid and their salts and esters, as well as combinations and mixtures thereof, in particular flurbiprofen and its salts and esters, preferably flurbiprofen; and/or
wherein the composition comprises flurbiprofen and/or its salts and/or esters, preferably flurbiprofen.

6. Composition for the use according to any of the preceding claims in the prophylactic and/or therapeutic topical treatment of viral diseases caused by SARS-CoV-2, preferably COVID-19.

7. Composition for the use according to any of the preceding claims,
wherein the composition is at least substantially free of n-propanol and/or at least substantially free of isopropanol, in particular at least substantially free of a primary alcohol.

8. Composition for the use according to any of the preceding claims,
wherein the composition comprises the anti-inflammatory agent, in particular benzydamine and/or its salts and/or esters, preferably benzydamine hydrochloride, in an amount ranging from 0.1 mg to 50 mg, in particular from 0.5 mg to 20 mg, preferably from 1 mg to 15 mg, more preferably from 2 mg to 10 mg, based on a dosage unit of the composition.

9. Composition for the use according to any of the preceding claims,
wherein the composition comprises the nonsteroidal anti-inflammatory drug, in particular flurbiprofen and/or its salts and/or esters, preferably flurbiprofen, in an amount ranging from 0.1 mg to 50 mg, in particular from 0.5 mg to 20 mg, preferably from 1 mg to 15 mg, more preferably from 2 mg to 10 mg, based on a dosage unit of the composition.

10. Composition for the use according to any of the preceding claims,
wherein octenidine and/or its salts and/or esters, preferably octenidine dihydrochloride, is administered with a daily dose in the range from 0.15 mg/day to 120 mg/day, in particular in the range from 0.15 mg/day to 120 mg/day, preferably in the range from 0.3 mg/day to 100 mg/day, particularly preferably in the range from 1.5 mg/day to 60 mg/day, more preferably in the range from 2.25 mg/day to 120 mg/day, most preferably in the range from 2.5 mg/day to 40 mg/day; and/or
wherein the composition for administering octenidine and/or its salts and/or esters, preferably octenidine dihydrochloride, is provided with a daily dose in the range from 0.15 mg/day to 120 mg/day, in particular in the range from 0.15 mg/day to 120 mg/day, preferably in the range from 0.3 mg/day to 100 mg/day, particularly preferably in the range from 1.5 mg/day to 60 mg/day, more preferably in the range from 2.25 mg/day to 120 mg/day, most preferably in the range from 2.5 mg/day to 40 mg/day.

11. Composition for the use according to any of the preceding claims,
wherein the composition is present and/or formed as a hard caramel; and/or
wherein the composition is present and/or formed as a lozenge, such that, upon dissolving, the composition releases a therapeutically effective amount of active ingredients and/or constituents, in particular octenidine and/or its salts and/or esters, preferably octenidine dihydrochloride, when the composition, in particular the solid dosage form, is administered and dissolved in the oral and pharyngeal cavity of a patient.

12. Composition for the use according to any of the preceding claims,
wherein the composition, in particular the solid dosage form, comprises the active ingredients and/or constituents in a solid matrix and/or mass, in particular in a matrix and/or mass based on sugars and/or sugar substitutes.

13. Composition for the use according to any of the preceding claims,
wherein the composition, in particular the solid dosage form, has a residual moisture content in the range from 0.1 % by weight to 10 % by weight, in particular in the range from 0.5 % by weight to 5 % by weight, preferably in the range from 1 % by weight to 3 % by weight, based on the composition, in particular the solid dosage form.

14. Composition for the use according to any of the preceding claims,
wherein the composition is used for exposure prophylaxis, in particular post-exposure prophylaxis and/or pre-exposure prophylaxis, against coronaviruses, preferably SARS-CoV-2; and/or
wherein the composition is used for the prevention of infection with coronaviruses, preferably SARS-CoV-2; and/or
wherein the composition is used to reduce the viral load of coronaviruses, preferably SARS-CoV-2, preferably to reduce the viral load in the oral, pharyngeal, and/or nasal cavity; and/or
wherein the composition is used in the pre-symptomatic stage and/or post-symptomatic stage, particularly in the pre-symptomatic stage, of viral diseases, preferably COVID-19, caused by coronaviruses, preferably SARS-CoV-2; and/or
wherein the composition is used in asymptomatic courses of viral diseases, preferably COVID-19, caused by coronaviruses, preferably SARS-CoV-2; and/or
wherein the composition is used in symptomatic courses of viral diseases, preferably COVID-19, caused by coronaviruses, preferably SARS-CoV-2.

15. Composition for the use according to any of the preceding claims,
wherein the composition, in particular octenidine and/or its salts and/or esters, preferably octenidine dihydrochloride, inactivates and/or kills the coronaviruses, preferably SARS-CoV-2; and/or
wherein the composition, in particular octenidine and/or its salts and/or esters, preferably octenidine dihydrochloride, inhibits and/or reduces and/or prevents the proliferation and/or replication of coronaviruses, preferably SARS-CoV-2.

## Revendications

1. Composition, en particulier composition pharmaceutique, pour une utilisation dans le traitement topique prophylactique et/ou thérapeutique de maladies virales provoquées par des coronavirus et pour une application topique sur les muqueuses de la cavité buccale, pharyngée et/ou nasale,
où la composition contient comme principe actif antiviral de l'octénidine et/ou ses sels et/ou esters, de préférence du dichlorhydrate d'octénidine,
où la composition se présente et/ou est formée comme une forme posologique solide sous la forme d'une pastille, où la pastille présente un poids total d'un intervalle de 0,5 g à 7 g, en particulier d'un intervalle de 1 g à 6 g, de préférence d'un intervalle de 2 g à 5 g, contenant par pastille :
- de l'octénidine et/ou ses sels et/ou esters, de préférence du dichlorhydrate d'octénidine, en une quantité d'un intervalle de 0,05 mg à 15 mg, en particulier d'un intervalle de 0,1 mg à 12,5 mg, de préférence d'un intervalle de 0,5 mg à 10 mg, de manière plus préférentielle d'un intervalle de 0,75 mg à 7,5 mg, de manière particulièrement préférée d'un intervalle de 1 mg à 5 mg;
- éventuellement, au moins un anesthésique local, en particulier de la lidocaïne, de préférence du chlorhydrate de lidocaïne, en une quantité d'un intervalle de 0,1 mg à 50 mg, en particulier d'un intervalle de 1 mg à 20 mg, de préférence d'un intervalle de 3 mg à 15 mg, de manière plus préférentielle d'un intervalle de 4 mg à 10 mg;
- éventuellement, au moins un acidifiant sous la forme d'un acide organique ou de ses sels ou esters, de préférence de l'acide tartrique et/ou de l'acide citrique, en une quantité d'un intervalle de 0,5 mg à 100 mg, en particulier d'un intervalle de 1 mg à 50 mg, de préférence d'un intervalle de 2 mg à 20 mg;
- éventuellement, au moins une huile d'anis, en particulier de l'huile d'anis étoilée, en une quantité d'un intervalle de 0,2 mg à 80 mg, en particulier d'un intervalle de 0,5 mg à 40 mg, de préférence d'un intervalle de 0,75 mg à 15 mg;
- éventuellement, au moins une huile essentielle, en particulier de l'huile de menthe poivrée, en une quantité d'un intervalle de 0,01 mg à 5 mg, en particulier d'un intervalle de 0,05 mg à 3 mg, de préférence d'un intervalle de 0,1 mg à 1 mg;
- éventuellement, au moins un extrait de café et/ou un extrait de thé, en particulier un extrait de café, en une quantité d'un intervalle de 0,05 mg à 100 mg, en particulier d'un intervalle de 0,5 mg à 50 mg, de préférence d'un intervalle de 1 mg à 20 mg;
- éventuellement, du propylène glycol en une quantité d'un intervalle de 0,05 mg à 50 mg, en particulier d'un intervalle de 0,5 mg à 20 mg, de préférence d'un intervalle de 1 mg à 10 mg;
- éventuellement, au moins une drogue mucilagineuse et/ou son extrait en une quantité d'un intervalle de 0,5 mg à 200 mg, en particulier d'un intervalle de 1 mg à 100 mg, de préférence d'un intervalle de 5 mg à 50 mg;
- éventuellement, au moins un autre principe actif et/ou ingrédient, en particulier un additif et/ou un excipient pharmaceutique, en particulier choisi dans le groupe constitué par les excipients de fabrication, les stabilisants, les émulsifiants, les antioxydants, les conservateurs, les humectants, les édulcorants et les sucrants, les agents d'ajustement du pH, les substances tampons du pH, les épaississants, les aromatisants, les antiseptiques, les colorants, les tampons, les odorants, les parfums, les agents de dilution, les liants, les mouillants et/ou les conservateurs, ainsi que leurs combinaisons et mélanges, en une quantité d'un intervalle de 0,1 mg à 500 mg, en particulier d'un intervalle de 0,5 mg à 250 mg, de préférence d'un intervalle de 1 mg à 100 mg;
- au moins un sucre et/ou au moins un substitut de sucre comme excipient; où le sucre est choisi dans le groupe constitué par le saccharose, le glucose, en particulier le dextrose, et le fructose et/ou où le substitut de sucre est choisi parmi les alcools de sucre, de préférence dans le groupe constitué par le mannitol, le xylitol, le sorbitol, l'isomalt, le sirop de maltitol, le lactitol, le leucrose, les fructooligosaccharides, les glucanes, le polyglucose, ainsi que leurs combinaisons et mélanges, de manière particulièrement préférée l'isomalt et/ou le mannitol, ainsi que leurs combinaisons et mélanges, de manière tout particulièrement préférée l'isomalt; et en quantités permettant de fournir et/ou d'obtenir le poids total de la pastille,
où la pastille est au moins essentiellement exempte de phénoxyéthanol.

2. Composition pour une utilisation selon la revendication 1,
où la composition contient l'octénidine et/ou ses sels et/ou esters sous la forme d'un sel de chlorhydrate, de préférence du dichlorhydrate d'octénidine.

3. Composition pour une utilisation selon la revendication 1 ou 2,
où l'anesthésique local est choisi dans le groupe de la lidocaïne et ses sels et esters, de préférence du chlorhydrate de lidocaïne.

4. Composition pour une utilisation selon l'une quelconque des revendications précédentes,
où la composition contient au moins un agent anti-inflammatoire, en particulier de la benzydamine et/ou ses sels et/ou esters, de préférence du chlorhydrate de benzydamine.

5. Composition pour une utilisation selon l'une quelconque des revendications précédentes,
où la composition contient au moins un agent anti-inflammatoire non stéroïdien, en particulier où l'agent anti-inflammatoire non stéroïdien est choisi dans le groupe constitué par le flurbiprofène, l'ibuprofène, le dexibuprofène, le naproxène, le kétoprofène, le dexkétoprofène, l'acide tiaprofénique, le diclofénac et l'acide acétylsalicylique et leurs sels et esters, ainsi que leurs combinaisons et mélanges, en particulier le flurbiprofène et ses sels et esters, de préférence le flurbiprofène; et/ou
où la composition contient du flurbiprofène et/ou ses sels et/ou esters, de préférence du flurbiprofène.

6. Composition pour une utilisation selon l'une quelconque des revendications précédentes, dans le traitement topique prophylactique et/ou thérapeutique de maladies virales provoquées par le SARS-CoV-2, de préférence du COVID-19.

7. Composition pour une utilisation selon l'une quelconque des revendications précédentes,
où la composition est au moins essentiellement exempte de n-propanol et/ou au moins essentiellement exempte d'isopropanol, en particulier au moins essentiellement exempte d'un alcool primaire.

8. Composition pour une utilisation selon l'une quelconque des revendications précédentes,
où la composition contient l'agent anti-inflammatoire, en particulier de la benzydamine et/ou ses sels et/ou esters, de préférence du chlorhydrate de benzydamine, en une quantité d'un intervalle de 0,1 mg à 50 mg, en particulier d'un intervalle de 0,5 mg à 20 mg, de préférence d'un intervalle de 1 mg à 15 mg, de manière plus préférentielle d'un intervalle de 2 mg à 10 mg, par rapport à une unité posologique de la composition.

9. Composition pour une utilisation selon l'une quelconque des revendications précédentes,
où la composition contient l'agent anti-inflammatoire non stéroïdien, en particulier du flurbiprofène et/ou ses sels et/ou esters, de préférence du flurbiprofène, en une quantité d'un intervalle de 0,1 mg à 50 mg, en particulier d'un intervalle de 0,5 mg à 20 mg, de préférence d'un intervalle de 1 mg à 15 mg, de manière plus préférentielle d'un intervalle de 2 mg à 10 mg, par rapport à une unité posologique de la composition.

10. Composition pour une utilisation selon l'une quelconque des revendications précédentes,
où l'octénidine et/ou ses sels et/ou esters, de préférence le dichlorhydrate d'octénidine, est administré à une dose journalière d'un intervalle de 0,15 mg/jour à 120 mg/jour, en particulier d'un intervalle de 0,15 mg/jour à 120 mg/jour, de préférence d'un intervalle de 0,3 mg/jour à 100 mg/jour, de préférence d'un intervalle de 1,5 mg/jour à 60 mg/jour, de manière particulièrement préférée d'un intervalle de 2,25 mg/jour à 120 mg/jour, de manière plus préférentielle d'un intervalle de 2,5 mg/jour à 40 mg/jour; et/ou
où la composition est destinée à l'administration d'octénidine et/ou de ses sels et/ou esters, de préférence de dichlorhydrate d'octénidine, à une dose journalière d'un intervalle de 0,15 mg/jour à 120 mg/jour, en particulier d'un intervalle de 0,15 mg/jour à 120 mg/jour, de préférence d'un intervalle de 0,3 mg/jour à 100 mg/jour, de préférence d'un intervalle de 1,5 mg/jour à 60 mg/jour, de manière particulièrement préférée d'un intervalle de 2,25 mg/jour à 120 mg/jour, de manière plus préférentielle d'un intervalle de 2,5 mg/jour à 40 mg/jour.

11. Composition pour une utilisation selon l'une quelconque des revendications précédentes,
où la composition se présente et/ou est formée comme un caramel dur; et/ou
où la composition se présente et/ou est formée comme une pastille, de sorte que la composition libère une quantité thérapeutiquement efficace de principes actifs et/ou de constituants, en particulier d'octénidine et/ou de ses sels et/ou esters, de préférence de dichlorhydrate d'octénidine, en suçant la pastille lorsque la composition, en particulier la forme posologique solide, est administrée et sucée dans la cavité buccale et pharyngée d'un patient.

12. Composition pour une utilisation selon l'une quelconque des revendications précédentes,
où la composition, en particulier la forme posologique solide, présente les principes actifs et/ou les constituants dans une matrice et/ou une masse solide, en particulier dans une matrice et/ou une masse à base de sucres et/ou de substituts de sucre.

13. Composition pour une utilisation selon l'une quelconque des revendications précédentes,
où la composition, en particulier la forme posologique solide, présente une humidité résiduelle d'un intervalle de 0,1 % en poids à 10 % en poids, en particulier d'un intervalle de 0,5 % en poids à 5 % en poids, de préférence d'un intervalle de 1 % en poids à 3 % en poids, par rapport à la composition, en particulier la forme posologique solide.

14. Composition pour une utilisation selon l'une quelconque des revendications précédentes,
où la composition est utilisée pour la prophylaxie d'exposition, en particulier la prophylaxie post-exposition et/ou la prophylaxie pré-exposition, à des coronavirus, de préférence SARS-CoV-2; et/ou
où la composition est utilisée pour la prophylaxie d'infection à des coronavirus, de préférence SARS-CoV-2; et/ou
où la composition est utilisée pour réduire la charge virale de coronavirus, de préférence SARS-CoV-2, de préférence pour réduire la charge virale dans la cavité buccale, pharyngée et/ou nasale; et/ou
où la composition est utilisée au stade présymptomatique et/ou au stade post-symptomatique, en particulier au stade présymptomatique, de maladies virales, de préférence du COVID-19, provoquées par des coronavirus, de préférence SARS-CoV-2; et/ou
où la composition est utilisée dans le cas d'évolutions asymptomatiques de maladies virales, de préférence du COVID-19, provoquées par des coronavirus, de préférence SARS-CoV-2; et/ou
où la composition est utilisée dans le cas d'évolutions symptomatiques de maladies virales, de préférence du COVID-19, provoquées par des coronavirus, de préférence SARS-CoV-2.

15. Composition pour une utilisation selon l'une quelconque des revendications précédentes,
où la composition, en particulier l'octénidine et/ou ses sels et/ou esters, de préférence le dichlorhydrate d'octénidine, inactive et/ou élimine les coronavirus, de préférence SARS-CoV-2; et/ou
où la composition, en particulier l'octénidine et/ou ses sels et/ou esters, de préférence le dichlorhydrate d'octénidine, inhibe et/ou réduit et/ou empêche la multiplication et/ou la réplication des coronavirus, de préférence SARS-CoV-2.
